# EUROPEAN PATENT SPECIFICATION

(11) **EP 4 138 799 B1**
(45) Date of publication and mention of the grant of the patent: **12.11.2025**
(21) Application number: 21721205.9
(22) Date of filing: 19.04.2021
(51) Int. Cl.: A61K 31/085, A61K 31/78, A61P 31/12, A61P 31/14, A61P 31/18, A61P 31/20, A61P 31/22

(54) **METHODS AND COMPOSITIONS FOR INHIBITING ENVELOPED VIRUSES USING LOW MOLECULAR WEIGHT HYDROPHOBICALLY MODIFIED POLYMERS**
VERFAHREN UND ZUSAMMENSETZUNGEN ZUR HEMMUNG VON UMHÜLLTEN VIREN MIT HYDROPHOB MODIFIZIERTEN POLYMEREN MIT NIEDRIGEM MOLEKULARGEWICHT
MÉTHODES ET COMPOSITIONS POUR INHIBER DES VIRUS ENVELOPPÉS FAISANT INTERVENIR DES POLYMÈRES HYDROPHOBIQUEMENT MODIFIÉS DE FAIBLE MASSE MOLÉCULAIRE

(30) Priority: 23.04.2020 US 202016856735
(43) Date of publication of application: 01.03.2023
(73) Proprietor: Kenvue Brands LLC, Summit, NJ 07901 (US)
(72) Inventor: BRUNING, Elizabeth, Skillman, New Jersey 08558 (US); CAPONE, Kimberly, Skillman, New Jersey 08558 (US); GANDOLFI, Lisa, Skillman, New Jersey 08558 (US); GEONNOTTI, Anthony Robert, Skillman, New Jersey 08558 (US); EKMAN-GUNN, Euen Thomas, Skillman, New Jersey 08558 (US); JOHNSON, Diana Roshek, Skillman, New Jersey 08558 (US); KIRCHNER, Frank J., Skillman, New Jersey 08858 (US); MOSES, Selina, Skillman, New Jersey 08558 (US); SANTORA, Delores, Skillman, New Jersey 08558 (US); WALTERS, Russel, Skillman, New Jersey 08558 (US); SUN, Frank C., Skillman, New Jersey 08558 (US)
(74) Representative: Carpmaels & Ransford LLP
(86) International application number: PCT/IB2021/053202
(87) International publication number: WO 2021/214624

(56) References cited:
- WO-A2-2005/074947
- US-A1- 2005 244 365
- US-A1- 2014 234 249

## Description

### Field of the Invention

The method of this invention relates to the use of low molecular weight hydrophobically modified polymers to inhibit the transmission of viruses known as "enveloped" viruses. It also relates to the use of compositions containing said low molecular weight hydrophobically modified polymers capable of inhibiting transmission of said viruses in methods of inhibiting the transmission of viruses.

### Background of the Invention

Infections due to enveloped viruses cause common diseases such as herpes simplex, HIV/AIDS, hepatitis B, influenza, chicken pox, shingles, small pox, and respiratory infections. While the seriousness of these diseases can range from moderately bothersome to life-threatening, these infections adversely affect the quality of life of its host and the personal, institutional and economic areas of our society. As a result, there have been substantial efforts to develop means to prevent viral infection and its spread. These efforts are complicated by viral diversity, the numerous means by which viruses are transmitted, including: direct contact, exchange of bodily fluids (e.g. saliva, sexual transmission, breast feeding), and aerosol transmission (e.g. coughing, sneezing, etc.) as well as the highly evolved measures by which viruses escape detection and/or eradication by their hosts. There have been numerous successes in the discovery and commercialization of antiviral agents administered to those who have been infected with a virus. However, these treatments often require medical prescriptions, have unwanted side effects, only work on a narrow range of viral types/strains, and/or have limited efficacy. Topically delivered antiviral treatments must also be non-irritating to the treated tissues, or risk increasing the risk of infection.

Therefore, cost effective and gentle agents with potent, broad-spectrum anti-viral activity which are capable of significantly reducing virus transmission would fill an unmet need in the antiviral armamentarium and help prevent the spread of viral infections, especially if mild properties of such agents could permit and encourage widespread, frequent usage due to superior compatibility with skin, eyes and other mucosal membranes.

Viruses have high mutation and replication rates; these properties allow rapid evolution in response to external selective pressures (i.e. drug), often leading to treatment resistance and relapse. The concern of resistance is especially salient when the antiviral compound targets a specific epitope on the virion. Due to high levels of viral genetic diversity, this narrow specificity also usually limits the range of viruses sensitive to the compound. Alternatively, other topical antiviral treatments, such as surfactants, target non-specific viral regions and are broadly effective at neutralizing diverse viruses, however, these are often irritating and toxic to human cells. Treatments that irritate tissues may result in an increased infection rate; damaging cellular membranes increases their permeability to some types of viral particles. Thus, a non-irritating yet highly effective means for eradicating viruses and significantly reducing their transmission potential would be highly desirable.

Most viruses (e.g., HIV and many animal viruses) have viral envelopes as their outer layer at the stage of their life-cycle when they are between host cells. Robertson et al. (March 1995). "Recombination in AIDS viruses." Journal of Molecular Evolution. 40 (3): 249-59. Some enveloped viruses also have a protein layer called a capsid between the envelope and their genome. Id. The envelopes are typically derived from portions of the host cell membranes (phospholipids and proteins), but include some viral glycoproteins. They may help viruses avoid the host immune system. Glycoproteins on the surface of the envelope serve to identify and bind to receptor sites on the host's membrane. The viral envelope then fuses with the host's membrane, allowing the capsid and viral genome to enter and infect the host.

The cell from which the virus itself buds will often die or be weakened and shed more viral particles for an extended period. The lipid bilayer envelope of these viruses is relatively sensitive to desiccation, heat, and detergents; therefore these viruses are easier to sterilize than non-enveloped viruses, have limited survival outside host environments, and typically transfer directly from host to host. Enveloped viruses possess great adaptability and can change in a short time in order to evade the immune system. Enveloped viruses can cause persistent infections.

Classes of enveloped viruses that contain human pathogens include, e.g., DNA viruses such as Herpesvirus, Poxviruses, Hepadnaviruses, Asfarviridae; RNA viruses such as Flavivirus, Alphavirus, Togavirus, Coronavirus, Hepatitis D, Orthomyxovirus, Paramyxovirus, Rhabdovirus, Bunyavirus, Filovirus; and Retroviruses such as HIV.

### COVID-19

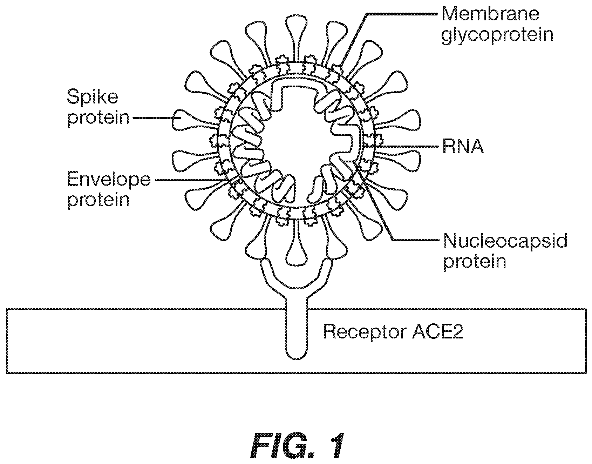

Coronaviruses (CoVs) are relatively large viruses containing a single-stranded positive-sense RNA genome encapsulated within a membrane envelope. The viral membrane is studded with glycoprotein spikes that give coronaviruses their crownlike appearance. (See Fig. 1, taken from Liu et al., Research and Development on Therapeutic Agents and Vaccines for COVID-19 and Related Human Coronavirus Diseases, ACS Cent. Sci. 2020, 6, 315-331). While coronaviruses infect both humans and animals, certain types of animals such as bats that host the largest variety of coronaviruses appear to be immune to coronavirus-induced illness. There are four classes of coronaviruses designated as alpha, beta, gamma, and delta. The betacoronavirus class includes severe acute respiratory syndrome (SARS) virus (SARS-CoV), Middle East respiratory syndrome (MERS) virus (MERS-CoV), and the COVID-19 causative agent SARS-CoV-2. Similar to SARS-CoV and MERS-CoV, SARS-CoV-2 attacks the lower respiratory system to cause viral pneumonia, but it may also affect the gastrointestinal system, heart, kidney, liver, and central nervous system leading to multiple organ failure. Current information indicates that SARSCoV-2 is more transmissible/contagious than SARS-CoV.

A number of studies have focused on elucidation of virus structure, virus transmission mechanisms/dynamics, as well as identification of antiviral agents and accurate diagnostics for virus detection. These trends reflect immense interest and desire from the scientific community, including both academic and industrial organizations as well as clinicians, to identify new methods to halt the progression of this epidemic disease and to prevent infection and transmission in the future.

COVID-19 is caused by SARS-CoV-2, a new type of coronavirus in the same genus as SARS-CoV and MERS-CoV. Viral proteins responsible for SARS-CoV-2 entry into host cells and replication are structurally similar to those associated with SARS-CoV. Thus, research and development on SARS and MERS may offer insights that would be beneficial to the development of therapeutic and preventive agents for COVID-19.

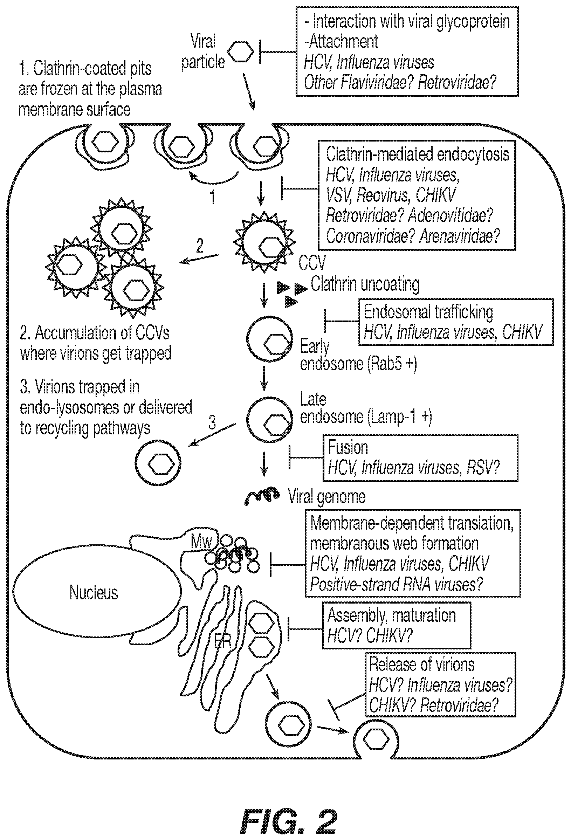

Arbidol, CAS No. 131707-23-8, which targets S protein/ACE2, is an inhibitor that may disrupt the binding of the viral envelope protein to host cells and prevent entry of the virus to the target cell has entered into clinical trials for treatment of COVID-19. See Liu et al. above and Fig. 2 below, taken from Blaising et al., Arbidol as a broad-spectrum antiviral: An update, Antiviral Research, 107 (2014) 84-94. See also Kadam et al., Structural basis of influenza virus fusion inhibition by the antiviral drug Arbidol, PNAS January 10, 2017 114 (2) 206-214.

The 2003 emergence of the severe acute respiratory disease coronavirus (SARS-CoV) demonstrated that CoVs are capable of causing outbreaks of severe infections in humans. A second severe CoV, Middle East respiratory syndrome coronavirus (MERS-CoV), emerged in 2012 in Saudi Arabia. More recently, COVID-19 identified in Wuhan, China, in December 2019, has proven particularly detrimental.

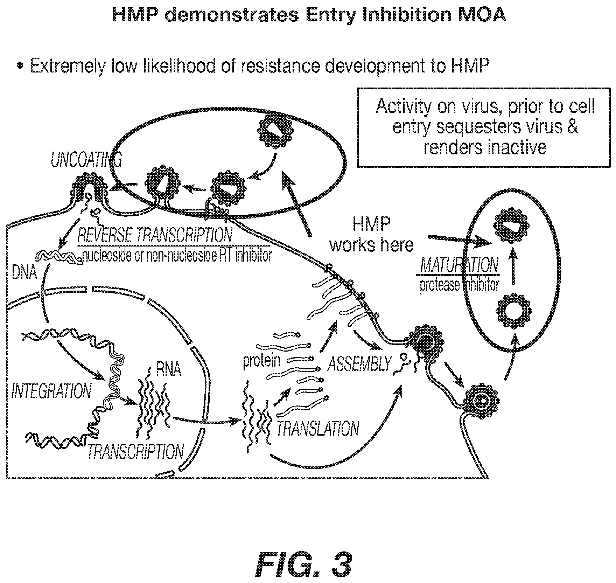

Given that the polymers of the invention have shown activity against enveloped viruses, it is expected that polymers of the invention may also show activity against COVID-19 by inhibiting entry of the virus in a host cell. See Fig. 3.

RetroVirox, San Diego, CA, has developed cell-based assays that can be used to evaluate experimental treatments against coronaviruses, including SARS-CoV-2. The Company provides testing with SARS-CoV-2 pseudoviruses to evaluate entry inhibitors against the novel coronavirus causative agent of COVID-19. The pseudovirus assay utilizes HIV pseudoviruses coated with the viral spike (S) protein of SARS-CoV-2 (Wuhan isolate). The assay, which recapitulates the mode of entry of the novel coronavirus, it can be used for, e.g., evaluate small-molecule entry inhibitors targeting the S viral protein, the ACE-2 viral receptor, or host proteases and other targets involved in SARS-CoV-2 viral entry.

U.S. Patents Nos. 7,803,403 and 8,025,902 to Johnson & Johnson Consumer Inc. disclose personal care compositions that contain a low molecular weight, non-cross linked, linear acrylic copolymer and at least one surfactant; and a method of cleansing using said personal care compositions.

U.S. Patents Nos. 8,343,902 and 8,329,626 to Johnson & Johnson Consumer Inc. disclose a skin cleansing composition that comprises a low molecular weight, non-crosslinked, linear acrylic copolymer and a non-ethoxylated anionic surfactant.

U.S. Patent No. 8,329,627 to Johnson & Johnson Consumer Inc. discloses a clear skin cleansing composition that comprises a low molecular weight, non-crosslinked, linear acrylic copolymer and a blend of at least two amphoteric surfactants.

U.S. Patent No. 8,293,845 to Lubrizol Corp. discloses a method for increasing the critical micelle concentration of a surfactant composition comprising including a linear hydrophobically modified (meth)acrylic polymer in said composition.

U.S. Patent No. 7,892,525 to Lubrizol Advanced Materials, Inc. discloses antiperspirant compositions that comprise a cationic hydrophobically modified polymeric gelling agent and an acidic antiperspirant compound.

U.S. Patent No. 9,068,148 to Lubrizol Advanced Materials, Inc. discloses an acrylic polymer blend that comprises at least one crosslinked acrylic copolymer and at least one acrylic linear, non-crosslinked polymer; a method for making the acrylic polymer blend; and method for thickening an aqueous composition comprising the acrylic polymer blend.

U.S. Patent No. 9,931,290 to Lubrizol Advanced Materials, Inc. discloses a surfactant composition that comprises a surfactant and a crosslinked acrylic copolymer; and a personal care cleansing composition comprising the surfactant composition.

U.S. Patent No. 10,517,806 to Ecolab USA Inc. claims a foaming antimicrobial dermal cleanser that comprises a cationic active ingredient; a cationic compatible surfactant; a foam boosting agent; a foam structure enhancing agent; a skin conditioning agent; and water. The reference claims a method of reducing bacterial, microbial, fungicidal, or viral population on a dermal tissue of a mammal comprising contacting the dermal tissue with the foaming antimicrobial dermal cleanser. The reference also discloses that cationic active ingredients are antimicrobial agents useful in the present invention and that the foam structure enhancing agent can be polyethyleneglycol. The reference discloses the use of S. aureus and Escherichia coli as test microbial cultures to test microbial efficacy of the formulas therein.

U.S. Patent No. 10,435,308 to Ecolab USA, Inc. claims a composition for improving oil removal from an oil/aqueous phase solution by foam fractionation that comprises an associative thickener; a surfactant comprising a sorbitan ester; and a viscoelastic surfactant, wherein the viscoelastic surfactant is a betaine, amine oxide, and/or ethoxylated fatty amine. The reference discloses that the composition may be used in, e.g., cleaning agents, cosmetics, pickles, aqueous pigment pastes, automotive finishes, industrial coatings, printing inks, lubricating greases, plaster paints and wall paints, textile coatings, pharmaceutical preparations, crop protection formulations, filler dispersions, adhesives, detergents, wax dispersions, polishes, auxiliaries for tertiary mineral oil production etc.

U.S. Published Application No. 20160262999 to Ecolab USA, Inc. claims an antimicrobial dermal concentrate that comprises a cationic active ingredient; a foam boosting surfactant; a foam boosting copolymer; a foam stabilizing structure; and water. The reference claims that the concentrate can be used to reduce bacterial, microbial, fungicidal or viral population on a dermal tissue of a mammal. The reference discloses that cationic active" is the ingredient that provides antimicrobial activity. The reference discloses that the concentrate may contain a skin conditioner such as polyethylene glycol.

Menachery et al., Pathogenic Influenza Viruses and Coronaviruses Utilize Similar and Contrasting Approaches To Control Interferon-Stimulated Gene Responses, American Society of Microbiology, 2014, 5(3): 1-11, discloses that influenza viruses and coronaviruses exhibit differences in terms of replication, immune stimulation, and overall lethality.

Li, Structure, Function and Evolution of Coronavirus Spike Proteins, Annu. Rev. Virul. 2016, 3(1):237-261, discusses the evolution of two critical functions of coronavirus spike proteins, receptor recognition and membrane fusion, in the context of the corresponding functions from other viruses and host cells.

Neutrogena Corp, Los Angeles, California, markets and sells a Neutrogena^{®} Ultra Gentle Daily Cleanser product that contains the use of potassium acrylates copolymer as a viscosity increasing agent.

Johnson & Johnson Consumer Inc. markets and sells products, including Johnson's Head to Toe Baby Wash; Johnson's Baby Moisture Wash; and Johnson's Baby Wipes that contain the use of potassium acrylates copolymer as a viscosity increasing agent.

Hand sanitizers are generally used to decrease infectious agents on the hands. They are available as liquids, gels, and foams. Alcohol-based versions and non-alcohol based versions are available. Alcohol-based versions typically contain some combination of isopropyl alcohol, ethanol (ethyl alcohol), or *n*-propanol, with versions containing 60% to 95% alcohol being the most effective. Care should be taken as they are flammable. Alcohol-based hand sanitizer works against a wide variety of microorganisms. Non-alcohol based versions, which typically contain benzalkonium chloride or triclosan, are less effective than alcohol-based ones.

In 2020, BlueWillow Biologics, Inc. launched NanoBio Project nasal antiseptic solution containing over-the-counter (OTC) monograph benzalkonium chloride. The product is applied by thoroughly swabbing the skin inside of each nostril.

US 2005/244365 discloses hydrophobically modified compounds of Formula I (hydrophobically modified cellulose) and Formula II (poly (methyl vinyl ether/maleic anhydride) with low molecular weight (500 daltons). Compounds of Formula I and II can be used for treating, or decreasing the frequency of transmission of a virus, such as human immunodeficiency virus (HIV-1) and herpes virus (HSV-1, HSV-2).

WO 2005/074947 discloses a composition comprising a cationic polymer poly (dimethylaminoethylmethacrylate (EUDRAGIT E) and in a concentration range of 0.1% to 5%, and a surfactant, benzalkonium chloride (HLB is 24) or benzethonium (HLB is 15), in a concentration range of 0.01% to 1%. The composition has an antiviral activity, which is defined as the capability of reducing the number of viral particles in an infected subject and/or reducing the likelihood of a subject exposed to potentially infective viral particles to contract a viral disease (viral infection is caused by a virus selected from the group consisting of influenza virus, rhinovirus, adenovirus, coronavirus, parainfluenza virus, respiratory syncytical virus, HIV virus, and herpes virus).

### SUMMARY OF THE INVENTION

This invention relates to an anti-viral composition for use in a method of inhibiting entry of enveloped viruses into cells comprising, consisting essentially of and consisting of contacting said viruses with said anti-viral composition comprising at least one low molecular weight hydrophobically modified polymer in an amount effective to inhibit entry of these viruses into cells, wherein said method comprises applying said anti-viral composition to infectable surfaces of a subject, said infectable surfaces comprising one or more of the group consisting of skin and mucosal tissue of a subject, wherein said low molecular weight hydrophobically modified polymer comprises a polymer derived from at least one first monomeric component selected from the group consisting of (meth)acrylic acid and at least one second monomeric component selected from the group consisting of one or more C₁ to C₉ alkyl (meth)acrylates, wherein the low molecular weight copolymer has a number average molecular weight of 100,000 Daltons or less, and wherein said composition is substantially free of anionic, cationic or amphoteric surfactant having a hydrophilic-lipophilic balance (hereinafter, "HLB") greater than 12.

The invention also relates to a method of inhibiting the transmission of viruses, wherein the method comprises applying to non-biological surfaces a composition comprising at least one low molecular weight hydrophobically modified polymers in an amount effective to inhibit entry of viruses into cells, wherein said composition is substantially free of anionic, cationic or amphoteric surfactant, wherein said low molecular weight hydrophobically modified polymer comprises a polymer derived from at least one first monomeric component selected from the group consisting of (meth)acrylic acid and at least one second monomeric component selected from the group consisting of one or more C₁ to C₉ alkyl (meth)acrylates, and wherein the low molecular weight copolymer has a number average molecular weight of 100,000 Daltons or less.

The invention also relates to a method of inhibiting the transmission of viruses, wherein the method comprises applying to ingestable surfaces a composition comprising at least one low molecular weight hydrophobically modified polymers in an amount effective to inhibit entry of viruses into cells, wherein said composition is substantially free of anionic, cationic or amphoteric surfactant, wherein said low molecular weight hydrophobically modified polymer comprises a polymer derived from at least one first monomeric component selected from the group consisting of (meth)acrylic acid and at least one second monomeric component selected from the group consisting of one or more C₁ to C₉ alkyl (meth)acrylates, and wherein the low molecular weight copolymer has a number average molecular weight of 100,000 Daltons or less.

Surprisingly, we have found that low concentrations of certain low molecular weight hydrophobically modified polymers known for their gentle properties are able successfully to inhibit entry of enveloped viruses into host cells and thus inhibit transmission of viruses to the hosts.

We believe that these polymers would not encounter or engender some of the historical problems with antiviral treatments, such as drug resistance, narrow breadth of neutralization and host cellular toxicity. The low molecular weight hydrophobically modified polymers useful in the methods and compositions of this invention are broadly active against several viral types and across multiple viral strains. Additionally, these polymers work through a non-specific mechanism of entry inhibition, thereby increasing their chances for inhibitory success and decreasing the likelihood of resistance. Furthermore, as these polymers are exceptionally gentle on mucosal tissues, they have little or no toxicity to human tissues.

Our bodies are challenged by viruses on a daily basis and our immune system, including our skin barrier, is designed to minimize the number of viruses that reach infectable surfaces. The low molecular weight hydrophobically modified polymers useful in the methods and compositions of this invention block the ability of the virus to bind to and/or enter cells, thereby reducing the probability that an infectious virus can reach a target cell and cause a systemic infection. Viral infection is partially the result of a stochastic process - the more viruses that come in contact with infectable cells, the more likely that tissue is to be infected - therefore, use of these polymers to block infectious viruses benefits the immune system, further reduces chances of infection and promotes general good health. The methods and compositions of this invention using low molecular weight hydrophobically modified polymers are surprisingly effective at reducing the number of infectious virions across a broad range of viral types and strains while remaining gentle and non-irritating to human tissues.

### DETAILED DESCRIPTION OF THE PREFERRED EMBODIMENTS

As used herein, the term "infectable surface" means a surface of a living animal the cells of which may be infected by a virus, including mammals such as human beings. Examples of such infectable surfaces are external skin tissues and mucosal tissues. Mucosal tissues include oral, ocular, nasal, vaginal and rectal tissue.

As used herein, the term "ingestible surface" refers to the surface of foods, including the surface of fruits and vegetables. As used herein, the term "hard surface" refers to surfaces found in the environment such as tables, chairs, walls, and other inanimate surfaces with which skin and/or mucosal tissue may come into contact and on which viruses may reside. The term "internal surface" refers to internal organ surfaces and internal tissues and fluids within the body of a living organism.

As used herein, the term "virus" means a small infectious agent that can replicate only inside the living cells or organisms. Virus particles contain the following parts: genetic material made from either RNA or DNA and a protein coat that protects the genetic material. In some cases, virus particles are surrounded by an envelope of lipids around the protein coat when the virus particles are outside a cell. Virus particles that contain such an envelope of lipids are referred to herein as "enveloped viruses". Enveloped viruses may include the following organisms: poxviridae including, but not limited to, molloscum contagiosum, chickenpox, smallpox and other pox viruses; Coronaviridae; Flaviviridae; Herpesviridae including herpes simplex virus 1 and herpes simplex virus 2; Retroviridae including Lentivirus including Human Immunodeficiency Virus.

As used herein, the term "surfactant" is a surface active agent, or a substance that, when dissolved in water or an aqueous solution, reduces its surface tension or the interfacial tension between it and another liquid.

As used herein, the term "inhibiting transmission" means one or more of the following: (i) impeding the entry of a virus into a host cell; (ii) substantially stopping the introduction of a virus from one individual, infectable surface or contact surface to another; and/or (iii) reducing damage to mucosal membranes such that the membranes retain their integrity and protect against infection by the virus.

As used herein, the hydrophilic-lipophilic balance ("HLB") is a measure of the degree to which a surfactant is hydrophilic or lipophilic, as determined by calculating values for different regions of the surfactant molecule in accordance with methods known to those of skill in the art.

Preferably, this invention relates to an anti-viral composition for use in a method of inhibiting entry of enveloped viruses into cells comprising, consisting essentially of and consisting of contacting said viruses with said anti-viral composition comprising, consisting essentially of and consisting of at least one low molecular weight hydrophobically modified polymer in an amount effective to inhibit entry of viruses into cells, said composition being substantially free of anionic, cationic or amphoteric surfactant having an HLB of greater than 12. The methods of this invention further include the application of the compositions set forth herein onto infectable surfaces.

Methods of the invention may include the application of the compositions onto ingestible surfaces. The methods further include contacting viruses with the anti-viral compositions of this invention.

The methods of this invention also include the application of the compositions of this invention to ingestible surfaces such as food as well as to hard surfaces into which skin and mucosal tissue might come into contact. As such, the presence of the compositions of this invention would work to inhibit entry of viruses present on ingestible and hard surfaces into cells contained on skin and mucosa and internal tissues and fluids.

Preferably, the compositions of this invention contain at least about 50% and preferably at least about 55% water. Alternatively, the compositions of this invention may contain at least 50% protic solvent, which may be selected from the following solvents: water, glycerine, urea, alkanols, acetic acid, formic acid and the like. More preferably, protic solvents useful in the compositions of this invention include formic acid, acetic acid, n-butanol, isopropanol, n-propanol, ethanol, methanol and the like.

Most preferably, the compositions of this invention are substantially free of surfactant having an HLB greater than about 12. Notwithstanding the foregoing, the compositions of this invention may additionally contain surfactants having an HLB of less than 12. Surfactants having HLB of greater than 12 may disrupt the cell membranes of the infectable surfaces, thus easing the ability of the viruses to enter and infect cells.

Preferably, the compositions useful in the methods of this invention have a Trans-Epithelial Permeability (hereinafter, "TEP"), as described below, of at least 6.

The compositions of this invention may be applied to infectable surfaces of a living entity including mammals, reptiles, birds, fish, bacteria, and the like. Infectable surfaces of these living entities may include, but are not limited to, skin, mucosal and internal tissues. Mucosal tissue includes, but is not limited to oral tissue, ocular tissue, nasal tissue, vaginal tissue, rectal tissue or a combination thereof. Importantly, the compositions and methods of this invention do not disrupt these biological surfaces or cause significant irritation of those surfaces.

### POLYMERIC MATERIAL

Examples of polymeric materials useful in the compositions and methods of this invention include low-molecular weight acrylic, other ethylenically-unsaturated polymers, polyesters, polycarbonates, polyanhydrides, polyamides, polyurethanes, polyureas, polyimides, polysulfones, polysulfides, combinations of two or more thereof, and the like. Examples of suitable low molecular weight acrylic polymers include hydrophobically-modified acrylic, polysaccharide, cellulose, starch polymers, combinations of two or more thereof, and the like. Suitable low molecular weight acrylic polymers include hydrophobically-modified acrylic polymers, as well as other acrylic polymers, any of which may be formed via solution, suspension, precipitation, dispersion, emulsion, inverse emulsion, microemulsion, micellar polymerization methods, and combinations of two or more thereof. The acrylic polymers for use in the present invention may be derived from any one or more monomers selected from the group consisting of (meth)acrylates, (meth)acrylamides, vinyl ethers, esters, and amides, allyl ethers, esters, amines, and amides, itaconates, crotonates, styrenics, and olefins. The acrylic polymers may be nonionic hydrophilic, nonionic hydrophobic, anionic, cationic, zwitterionic, nonassociative macromer, associative macromer, or multifunctional/crosslinking.

As used herein the term "low molecular weight" polymer refers to a polymer having a number average molecular weight (Mₙ) of about 100,000 daltons or less as measured by gel permeation chromatography (GPC) calibrated with a poly(methyl methacrylate) (PMMA) standard. In certain preferred embodiments, low-molecular weight polymers are those having molecular weight ranges of from about 5,000 to about 80,000 Mₙ, more preferably from about 10,000 to about 50,000 Mₙ, and more preferably between about 15,000 and 40,000 Mₙ.

Certain hydrophobically-modified polymers and methods of making such polymers are described in U.S. Pat. No. 6,433,061, issued to Marchant et al.. The polymeric materials useful in the composition of this invention are preferably non-crosslinked, linear acrylic copolymers that are very mild to the skin and mucosa. These non-crosslinked, linear polymers are of low molecular weight having a number average molecular weight of 100,000 daltons or less as measured by gel permeation chromatography (GPC) calibrated with a poly(methyl methacrylate) (PMMA) standard (as used herein, unless otherwise specified, all number average molecular weights (Mₙ) refer to molecular weight measured in such manner). Thus, the polymeric material functions as a copolymeric compound. The copolymeric compound is polymerized from at least two monomeric components. The second monomeric component is hydrophobically modified (relative to the first monomeric component).

Thus, the hydrophobically modified polymers useful in the compositions and methods of this invention comprise, consist essentially of and consist of a low molecular weight, non-crosslinked, linear acrylic copolymer derived from at least one first monomeric component selected from the group consisting of (meth)acrylic acid and at least one second monomeric component selected from the group consisting of one or more C₁ to C₉ alkyl (meth)acrylates, wherein the low molecular weight copolymer has a number average molecular weight of about 100,000 daltons or less.

Exemplary first monomeric components include (meth)acrylic acid, itaconic acid, citraconic acid, maleic acid, fumaric acid, crotonic acid, aconitic acid, and mixtures thereof. Exemplary second monomeric components include ethyl (meth)acrylate, butyl (meth)acrylate, 2-ethylhexyl (meth)acrylate, vinyl formate, vinyl acetate, 1-methylvinyl acetate, vinyl propionate, vinyl butyrate, vinyl 2-ethylhexanoate, vinyl pivalate, vinyl neodecanoate, and mixtures thereof. As used herein, the terms "(meth)acrylic" acid and "(meth)acrylate" are meant to include the corresponding methyl derivatives of acrylic acid and the corresponding alkyl acrylate. For example, "(meth)acrylic" acid refers to acrylic acid and/or methacrylic acid and "(meth)acrylate" refers to alkyl acrylate and/or alkyl methacrylate.

More preferably, said first monomeric component is selected from the group consisting of (meth)acrylic acid and said second monomeric component is selected from the group consisting of at least one C₁ to C₉ alkyl (meth)acrylate.

The non-crosslinked, linear acrylic copolymer compounds useful in the compositions and methods of this invention can be synthesized via free radical polymerization techniques known in the art. In one aspect of the invention, the amount of the first monomeric component to the second monomeric component utilized ranges from about 20:80 wt. % to about 50:50 wt. %, based on the total weight of all of the monomers in the polymerization medium. In another aspect the weight ratio of the first monomeric component to the second monomeric component is about 35:65 wt. %, and in a further aspect the weight ratio of first monomeric component to second monomeric component is about 25:75 wt. %, all based on the total weight of all monomers in the polymerization medium.

Methods of synthesizing the polymers useful in the compositions and methods of this invention may be found in U.S. 6,433,061.

The linear copolymeric materials useful in the methods and compositions of this invention preferably have a viscosity of 500 mPa·s or less (Brookfield RVT, 20 rpm, spindle no. 1) at a 5 wt. % polymer solids concentration in deionized water and neutralized to pH 7 with an 18 wt. % NaOH solution. The viscosity can range from about 1 to about 500 mPa·s in another aspect, from about 10 to about 250 mPa·s in a further aspect, and from about 15 to about 150 mPa·s in a still further aspect.

Preferably, the low molecular weight, non-crosslinked linear acrylic copolymer present in the compositions and methods of this invention is potassium acrylates copolymer.

The low molecular weight hydrophobically modified polymers useful in the compositions and methods of this invention are present in said compositions in amounts that are effective to inhibit substantially the entry of enveloped viruses into cells and/or to inhibit virus transmission to cells. Accordingly, the compositions and methods of this invention inhibit virus entry into said cells and results in the reduction of the potential for viral infection. Preferably, they should be present in the compositions of this invention in an amount of from about 0.00005% to about 10% percent by weight of the composition. Even more preferably, they should be present in the amount of from about 0.00005% to about 3% by weight of the composition. More preferably, the low molecular weight hydrophobically modified polymers are present in an amount of from about 0.00005% to about 0.5 percent by weight of the composition. Most preferably, the low molecular weight hydrophobically modified polymers are present in an amount of from about 0.00005% to about 0.01% percent by weight of the composition.

The compositions of this invention may be in the form of a lotion or liquid capable of being applied on the surface of the skin or on an inanimate surface that can contain viruses or bacteria. It may also be a composition which is applied to a mucosal surface such as the surfaces of the nasal cavity or vaginal cavity and can be used as a vaginal microbicide. These types of composition may be more viscous and may be based on a gel formation. The compositions of this invention may be coated onto an absorbent article such as a vaginal or nasal tampon for placement in contact with mucosal surfaces to inhibit viruses in such biologic environments. The compositions of this invention may also be formulated in such a delivery form that they may be injected into the body at appropriate sites where viruses may reside on internal surfaces.

The compositions of this invention may be made into a wide variety of product types that include but are not limited to liquids, lotions, creams, gels, sticks, sprays, shaving creams, ointments, cleansing liquid washes and solid bars, shampoos, pastes, powders, mousses, wipes, patches, wound dressing and adhesive bandages, hydrogels and films. These product types may contain several types of cosmetically acceptable topical carriers including, but not limited to solutions, emulsions (e.g., microemulsions and nanoemulsions), gels, solids and liposomes. The following are non-limiting examples of such carriers. Other carriers may be formulated by those skilled in the art of formulating such product types.

Preferred compositions of the invention include polymer containing gels; polymer containing drops, including, e.g., eye drops; polymer containing contact lens solutions; polymer containing sprays, e.g., face/body sprays, nasal sprays, and mouth and throat sprays; and polymer containing inhalants.

The compositions of the invention may also be used as a coating on or in personal protective equipment. Personal protective equipment, which is commonly referred to as "PPE", is any equipment worn to minimize exposure to a variety of hazards. Examples of PPE include full body suits, gloves, gowns, masks, respirators and eye and foot protection.

The topical compositions useful in the methods of this invention may be formulated as solutions. Solutions preferably contain an aqueous solvent (e.g., from about 50% to about 99.99% or from about 90% to about 99% of a cosmetically acceptable aqueous solvent).

Topical compositions useful in the methods of this invention may be formulated as a solution containing an emollient. Such compositions preferably contain from about 2% to about 50% of an emollient(s). As used herein, "emollients" refer to materials used for the prevention or relief of dryness, as well as for the protection of the skin. A wide variety of suitable emollients is known and may be used herein. Sagarin, Cosmetics, Science and Technology, 2nd Edition, Vol. 1, pp. 32-43 (1972) and the International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "ICI Handbook") contain numerous examples of materials for use in the compositions and methods of this invention.

A lotion may also be made from such a solution. Lotions preferably contain from about 1% to about 20% (more preferably, from about 5% to about 10%) of an emollient(s) and from about 50% to about 90% (more preferably, from about 60% to about 80%) of water.

Another type of product that may be formulated from a solution is a cream. A cream preferably contains from about 5% to about 50% (more preferably, from about 10% to about 20%) of an emollient(s) and from about 45% to about 85% (more preferably from about 50% to about 75%) of water.

Yet another type of product that may be formulated from a solution is an ointment. An ointment may contain a simple base of animal or vegetable oils or semi-solid hydrocarbons. An ointment may preferably contain from about 2% to about 10% of an emollient(s) plus from about 0.1% to about 2% of a thickening agent(s). A more complete disclosure of thickening agents or viscosity increasing agents useful herein may be found in Sagarin, Cosmetics, Science and Technology, 2nd Edition, Vol. 1, pp. 72-73 (1972) and the ICI Handbook pp. 1693-1697.

The topical compositions useful in the methods of this invention may also be formulated as emulsions. If the carrier is an emulsion, preferably from about 1% to about 10% (e.g., from about 2% to about 5%) of the carrier contains an emulsifier(s). Emulsifiers may be nonionic, anionic or cationic. Suitable emulsifiers are set forth in, for example, U.S. Patent No. 3,755,560, U.S. Patent No. 4,421,769, McCutcheon's Detergents and Emulsifiers, North American Edition, pp. 317-324 (1986) and the ICI Handbook, pp.1673-1686.

Lotions and creams may also be formulated as emulsions. Preferably such lotions contain from 0.5% to about 5% of an emulsifier(s). Such creams would preferably contain from about 1% to about 20% (more preferably, from about 5% to about 10%) of an emollient(s); from about 20% to about 80% (more preferably, from 30% to about 70%) of water; and from about 1% to about 10% (more preferably, from about 2% to about 5%) of an emulsifier(s).

Other compositions useful in the methods of this invention include gels and liquid compositions that may be applicable to mucosal surfaces for inhibiting viral transmission. Mucosal surfaces include but are not limited to the vagina, rectum, nasal passages, mouth and throat. Preferably, such compositions should include at least one polyhydric alcohol, including glycerin, polyethylene glycol, propylene glycol, sorbitol or a combination thereof. Other polyhydric alcohols know to those of ordinary skill in the art may be used in the compositions and methods of this invention, including polyethylene glycols ranging from molecular weight of from about 300 to about 1450. Preferably, there should be from about 0.1 to about 50% by weight of glycerin and from about 2 to about 40% by weight of propylene glycol.

The mucosal compositions of this invention should also contain one or more water-soluble cellulose-derived polymers. Preferably, such polymers should be a cellulose gum such as one or more hydroxyalkylcellulose polymer. More preferably, the hydroxyalkylcellulose polymer should be one or more of hydroxyethylcellulose, hydroxymethylcellulose, hydroxypropylcellulose, hydroxypropylmethylcellulose and the like. Preferably, the cellulose-derived polymer should be present in the compositions of this invention in the amount of from about 0.1 to about 2% by weight of the composition.

The compositions of this invention intended for vaginal use may also contain one or more spermicides including but not limited to nonoxynol-9 and the like. Although such spermicides may be classified as surfactants, they generally have an HLB of less than 16 and are not useful as or in cleansing compositions and do not foam.

Preferably, an inorganic base may be used to adjust the pH of the composition to be compatible with the vaginal, oral or rectal mucosa. Potassium hydroxide or another alkali metal or alkaline earth metal base may be useful to provide the appropriate pH. Of course, any other physiological acceptable base may also be used in this manner. From about 0.05 to about 5% by weight inorganic base is preferably used.

The compositions of this invention may be prepared in accordance with those methods and processes known to those of skill in the art, or in accordance with the methods of preparation of this invention. For example, water-soluble components such as glycerin, propylene glycol, sorbitol, inorganic base, preservatives, and the like may be dissolved in water and to that combination cellulose-derived polymers may be added. Another method of preparation is mixing all the ingredients into a slurry without water, and then adding the slurry to water.

The composition is substantially free of anionic, cationic, or amphoteric surfactants.

Included in a liquid or lotion formation of the composition may be water, oils, preservatives, emulsifiers, viscosity enhancers, emollients, electrolytes, fragrance, buffers, pH modifiers, skin protectants, metal ion sequestrants and the like.

The compositions of this invention may be useful in formulating hand and/or body washes, fruit and/or vegetable washes, ingestible compositions, suppositories, nasal sprays, post-surgical tampons and the like, which may be applied to surfaces or placed in the body to inhibit transmission of viruses. The compositions of this invention may be coated onto an absorbent article such as a vaginal tampon or nasal swab for placement in contact with mucosal surfaces to inhibit viruses in such biologic environments.

### METHODS

There are various testing methods that have been employed herein to evaluate different aspects of the methods and compositions of this invention and their effects upon skin, mucosa and viruses when exposed to the compositions of the invention. The Trans-Epithelial Permeability ("TEP") test is used in the instant methods and in the following Examples. The TEP test is used to determine the degree to which a composition causes irritation to the skin or mucosa.

### Trans-Epithelial Permeability Test ("TEP Test"):

Irritation to the eyes and/or skin expected for a given formulation is measured in accordance with the Invittox Protocol Number 86 (May 1994), the "Trans-epithelial Permeability (TEP) Assay" and set forth in U.S. Patent No. 7,157,414. In general, the ocular and/or skin irritation potential of a product may be evaluated by determining its effect on the permeability of a cell layer, as assessed by the leakage of fluorescein through the layer. Monolayers of Madin-Darby canine kidney (MDCK) cells are grown to confluence on microporous inserts in a 24-well plate containing medium or assay buffer in the lower wells. The irritation potential of a product is evaluated by measuring the damage to the permeability barrier in the cell monolayer following a 15 minute exposure to dilutions of the product. Barrier damage is assessed by the amount of sodium fluorescein that leaks through to the lower well after 30 minutes, as determined spectrophotometrically.

The fluorescein leakage is plotted against the concentration of test material to determine the EC₅₀ (the concentration of test material that causes 50% of maximum dye leakage, i. e., 50% damage to the permeability barrier). Higher scores are indicative of milder formulas.

Exposure of a layer of MDCK cells grown on a microporous membrane to a test sample is a model for the first event that occurs when an irritant comes in contact with the eye. In vivo, the outermost layers of the corneal epithelium form a selectively permeable barrier due to the presence of tight junctions between cells. On exposure to an irritant, the tight junctions separate, thereby removing the permeability barrier. Fluid is imbibed to the underlying layers of epithelium and to the stroma; causing the collagen lamellae to separate, resulting in opacity. The TEP assay measures the effect of an irritant on the breakdown of tight junctions between cells in a layer of MDCK cells grown on a microporous insert. Damage is evaluated spectrophotometrically, by measuring the amount of marker dye (sodium fluorescein) that leaks through the cell layer and microporous membrane to the lower well.

### Virucidal Suspension Test:

Summary of experiment: A Virucidal Suspension Test (In-Vitro Time-Kill method) may be used to evaluate the virucidal properties of the hydrophobically-modified polymers useful in the compositions and methods of this invention when challenged with Herpes Simplex Virus type1 (HSV-1) strain HF (ATCC#VR-260).

The raw material, for example, potassium acrylates copolymer, may be supplied from the vendor as a 30% stock solution. The challenge viral strain for testing against HSV-1 is Herpes Simplex Virus type 1 strain HF (ATCC# VR-260). Host cells should be prepared as follows: Vero cells (ATCC#CCL-81) are maintained as monolayers in disposable cell culture labware and used for the Virucidal Suspension Test of HSV-1 strain HF. Prior to testing, host cell cultures are seeded onto the appropriate cell culture plates. Cell monolayers should be 100% confluent and less than 48 hours old before inoculation with the virus. The growth medium (GM) and maintenance medium (MM) was 1X Minimum Essential Medium (MEM) with appropriate supplements.

The test virus is prepared as follows: The HSV-1 strain HF from BSL1 high titer virus stock may be used for this study. On the day of use, aliquots of the stock virus are removed from a -70°C freezer and thawed prior to use in testing. Test product compositions of this invention may be prepared as follows: 5.0ml of the test product 30% solution is added to 4.5 ml Phosphate Buffered Saline with 0.5 ml of Sodium Hydroxide. The mixture is vortexed and pH measured using pH strips. The pH of the solution (15% v/v of the test product) should be 6.0 to 6.5. The test product is diluted to a 90% (v/v) concentration due to virus inoculation and simulation of virus inoculation. The test product is evaluated at a 13.5% (v/v) concentration. The percent and log₁₀ reductions from the initial population of the viral strain are determined following exposure to the test product for 15 minutes, 30 minutes, and 1 hour. Plating is performed in four replicates. Testing is performed in accordance with Good Laboratory Practices, as specified in 21 CFR Part 58.

### Neutralization Test:

The Neutralization Test may be performed prior to the Virucidal Suspension Test. Maintenance medium (hereinafter, "MM") is added to a sample of the test product, in simulation of the virus inoculum, to achieve the 90% (v/v) concentration of the test product. The mixture is added to the appropriate amount of neutralizer and mixed thoroughly. An aliquot of the neutralizer/MM/product is discarded and replaced with the test virus. Subsequent 10-fold dilutions of the neutralized product are made in MM. The dilutions are plated in four replicates.

To perform the Cytotoxicity Test, MM is added to a sample of the test product, in simulation of the virus inoculum. The mixture is added to the appropriate amount of neutralizer and mixed thoroughly. Subsequent 10-fold dilutions of the neutralized product are made in MM. The dilutions are plated in four replicates.

The evaluation of the Neutralizer Toxicity to the test virus may be performed as follows:
Virus Control #1 is used to determine if the neutralizer had a significant inhibitory effect upon the test virus as well as to define the titer of the test virus to be treated with neutralizer as in the Neutralization Test. The test virus is diluted in the neutralizer and subsequent dilutions are performed in MM. Each dilution is plated in four replicates. Virus Control #2 is used to determine the titer of the test virus when not treated with the neutralizer. 10-fold dilutions of the test virus are made in MM. Each dilution is plated in four replicates. Cell Culture Control: Intact cell culture monolayers serve as the control of cell culture viability. The GM is replaced by MM in all cell culture control wells.

The plates are incubated in a CO₂ incubator for 5 to 14 days at 37°C ± 2°C. Cytopathic/cytotoxic effects are monitored using an Inverted Compound Microscope.
*Virucidal Test:* The appropriate amount of the test virus is added to a sample of the test product and mixed thoroughly to achieve the 90% (v/v) concentration of the product. The test virus is exposed to the test product for 15 minutes, 30 minutes, and 1 hour, timed using a calibrated minute/second timer. Immediately after each exposure, the test virus/product suspensions are neutralized and diluted 1:10 in MM. Each dilution is plated in four replicates.
*Virus Control:* The appropriate amount of the test virus is treated with neutralizer the same way as in the Virucidal Test and subsequently diluted 1:10 in MM. Each dilution is plated in four replicates.
*Cytotoxicity Control:* Aliquots of MM are added to a sample of the test product to simulate the virus inoculums. The MM/product mixture is neutralized and diluted 1:10 in MM. Each dilution is plated in four replicates.
*Neutralization Control:* Aliquots of MM are added to a sample of each test and comparison product to simulate the virus inoculums. The MM/product mixture is neutralized and diluted 1:10 in MM. Approximately 100-1000 infectious units of the virus are added to a sample of the neutralized product. The dilutions are plated in four replicates.
*Cell culture Control:* Intact cell culture monolayers serve as the control of cell culture viability. The GM is replaced by MM in all cell culture control wells.The plates are incubated in a CO₂ incubator for 5 to 14 days at 37°C± 2°C. Cytopathic/cytotoxic effects are monitored using an Inverted Compound Microscope.

### Test Acceptance Criteria

A valid test requires: 1) at least a 4 log₁₀ of TCID₅₀ (Tissue Culture Infective Dose) recovered from the Virus Control; 2) cells in the cell culture control wells remain viable and attached to the bottom of the well; 3) the medium remain free of contamination in all wells of the plate; 4) when cytotoxicity is evident, at least a 3 log₁₀ reduction in titer demonstrated beyond the cytotoxic level, and 5) the test product fully neutralized immediately after the timed exposure such that the virus infectivity is not affected.

The following protocols may be used to determine the activity of the inventive embodiments against HIV-1, Hepatitis B, Influenza, Adenovirus, and Rhinovirus Protocol.

### Evaluation of Activity Against HIV-1_{IIIB} in CEM-SS Cells

Fifty microliters (50µL) of CEM-SS cells at a density of 2.5x10³ cells/well in 10% complete Roswell Park Memorial Institute Medium ("RPMI")-1640 (10% FBS with 1% L-glutamine and 1% Penicillin/Streptomycin, available commercially from Invitrogen located in Carlsbad, California) media are plated in a 96-well round bottom plate. One-hundred microliters (100 µL) of each polymer at 6 concentrations are added in triplicate followed by 50 µL of HIV-1_{IIIB} at a pre-determined titer. The cultures are incubated for 6 days at 37°C/5% CO₂. Following the incubation, the cells are stained with XTT for evaluation of compound efficacy and cellular toxicity, as described below. AZT is evaluated in parallel as an assay positive control compound.

### XTT Staining for Cell Viability and Compound Cytotoxicity:

TC₅₀ values for the test materials are derived by measuring the reduction of the tetrazolium dye XTT (2,3-bis(2-methoxy-4-nitro-5-sulfophenyl)-5-[(phenylamino)carbonyl]-2H-tetrazolium hydroxide). XTT in metabolically active cells is metabolized by the mitochondrial enzyme Nicotinamide adenine dinucleotide phosphate oxidase ("NADPH") to a soluble formazan product. XTT solution is prepared daily as a stock of 1 mg/ml in RPMI-1640 without additives. Phenazine methosulfate (PMS) solution is prepared at 0.15 mg/ml in DPBS and stored in the dark at -20°C. XTT/PMS stock is prepared immediately before use by adding 40 µL of PMS per ml of XTT solution. Fifty µL (50 µL) of XTT/PMS is added to each well of the plate and the plate incubated for 4 hours at 37°C. The 4 hour incubation has been empirically determined to be within the linear response range for XTT dye reduction with the indicated numbers of cells for each assay. The plates are sealed and inverted several times to mix the soluble formazan product and the plate is read at 450 nm (650 nm reference wavelength) with a Molecular Devices SpectraMax Plus 384 96 well plate format spectrophotometer.

### Evaluation of HIV-1 Activity in Human Peripheral Blood Mononuclear Cells ("PBMCs"):

The leukophoresed blood cells are washed several times with Dulbeccos's Phosphate Buffered Saline (DPBS). After washing, the leukophoresed blood is diluted 1:1 with DPBS and layered over 15 ml of Ficoll-Hypaque density gradient in a 50 ml conical centrifuge tube. These tubes are then centrifuged for 30 min at 600 X g. Banded PBMCs are gently aspirated from the resulting interface and subsequently washed three times with DPBS by low speed centrifugation. After the final wash, cells are enumerated by Trypan Blue dye exclusion and re-suspended at 1 x 10⁶ cells/ml in RPMI 1640 with 15% Fetal Bovine Serum (FBS), 2 mmol/L L-glutamine, 2 µg/ml PHA-P, 100 Units/ml penicillin and 100 µg/ml streptomycin and allowed to incubate for 48 to 72 hours at 37°C. After incubation, the PBMCs re centrifuged and resuspended in tissue culture medium (RPMI 1640 with 15% FBS, 2 mmol/L L-glutamine, 100 U/ml penicillin, 100 µg/ml streptomycin and 3.6 ng/ml recombinant human IL-2). The cultures are then maintained until use by half culture volume change with fresh IL-2 containing tissue culture medium every 3 days. Assays are initiated with PBMCs that have been induced to proliferate with PHA-P for 72 hours. For the PBMC assay, PHA-P stimulated PBMCs from two donors are pooled together to minimize the variability that occurs when cells from individual donors are used, resuspended in fresh tissue culture medium at 1 x 10⁶ cells/ml and plated in the interior wells of a 96-well round bottom microtiter plate at 50 µL/well. One-hundred microliters (100 µL) of 2-times the concentration of polymer-containing medium is transferred to designated wells of the round-bottom 96-well plate containing the cells in triplicate. Immediately following addition of the polymer to the wells, 50 µL of a predetermined dilution of virus is added, and mixed well. After 7 days in culture at 5% CO₂/37°C HIV-1 replication is quantified by the measurement of cell-free HIV-1 RT activity in the tissue culture supernatant as described below. Cytotoxicity is evaluated using the tetrazolium dye XTT as described above.

### Evaluation of Chronic HIV-1 Replication Inhibition:

CEM-SS cells chronically infected with HIV-1_{IIIB} are added to a 96 well microtiter plate at a density of 2.5x10³ cells per well in a 100 µL volume. The compound(s) are diluted serially so that a total of six concentrations are evaluated. One hundred microliters (100 mL) of each concentration is added in triplicate to the cells. The plates are incubated at 37°C/5% CO₂ for 6 days. Following incubation, cell-free supernatant samples are collected from each well of the 96 well plate and analyzed for reverse transcriptase (RT) activity. The plate is stained with XTT tetrazolium dye for measurement of cell viability.

### Reverse Transcriptase Activity Assay:

Reverse transcriptase is measured in cell-free supernatants using a standard radioactive incorporation polymerization assay. Tritiated thymidine triphosphate (TTP) is purchased at 1 Ci/ml and 1 µL was used per enzyme reaction. Poly rA and oligo dT are prepared at concentrations of 0.5 mg/ml and 1.7 Units/ml, respectively, from a stock solution which is kept at -20°C. The RT reaction buffer is prepared fresh on a daily basis and consists of 125 µL of 1 M EGTA, 125 µL of dH₂O, 125 µL of 20% Triton X-100, 50 µL of 1 M Tris (pH 7.4), 50 µL of 1 M DTT, and 40 µL of 1 M MgCl₂. For each reaction, 1 µL of TTP, 4 µL of dH₂O, 2.5 µL of rAdT and 2.5 µL of reaction buffer are mixed. Ten microliters (10 µL) of this reaction mixture is placed in a round bottom microtiter plate with 15 µL of virus containing supernatant. The plate is incubated at 37°C in a humidified incubator for 60 to 90 minutes. Following the incubation, 10 µL of the reaction volume is spotted onto a DEAE filter mat in the appropriate plate format, washed 5 times for 5 minutes each in a 5% sodium phosphate buffer, 2 times for 1 minute each in distilled water, 2 times for 1 minute each in 70% reagent alcohol, and then air dried. The dried filtermat is placed in a plastic sleeve and 4 ml of Opti-Fluor O was added to each sleeve. Incorporated radioactivity is quantified utilizing a Wallac 1450 Microbeta Trilux liquid scintillation counter.

### Evaluation of Cell to Cell Virus Transmission Inhibition

Uninfected CEM-SS cells are plated in a 96-well flat bottom plate at a density of 1x10⁵ cells per well in a total volume of 50 µL. The chronically HIV-1_{IIIB} infected CEM-SS cells are added at cell densities ranging from 1x10⁵ cells per well to 1x10⁰ cells per well in a volume of 50 µL. The test compounds are diluted serially to achieve the test concentrations and are added in triplicate wells in a volume of 100 µL. The plate is incubated at 5% CO₂/37°C for 48 hours. Following the incubation, the number of syncytia per well is counted. Following an additional 24 hour incubation, cell-free supernatant samples from each well of the 96 well plates are analyzed for reverse transcriptase (RT) activity as described above.

### Evaluation of HIV-1_{BaL} Entry Inhibition in TZM-bl-FcRI Cells

Twenty-four hours prior to compound exposure, TZM-bl-FcRI cells are plated in a 96-well flat bottom plate at 1x10⁴ cells per well in a 100 µL. Following an incubation at 5% CO₂/37°C, 50 µL of compound diluted serially is added to the cells in triplicate 10 to 15 minutes prior to the addition of HIV-1_{BaL}. HIV-1_{BaL} is diluted to pre-determined titer and added to the efficacy plates in a volume of 50 µL. Media is added to the toxicity plates in the same volume. Following a two hour incubation at 5% CO₂/37°C the cultures are washed to remove residual virus and compound. The plates are incubated at 5%CO₂/37°C for an additional 48 hours at which time the efficacy plates are evaluated using a chemiluminescent substrate (Gal Screen, Tropix) and toxicity is evaluated using XTT as described above.

### Evaluation of Compound Efficacy Using Chemiluminescence Detection

All media is removed from the efficacy plates and replaced with 50 mL of DPBS. Fifty microliters (50 µL) of Gal-Screen substrate diluted 1:25 in Gal Screen Buffer is added to all wells of the plate. The plate is incubated for 90 minutes at room temperature. Following the incubation, the contents of the wells is transferred to a clear bottom plate. The plate is covered and chemiluminescence detected using a Microbeta Scintillation Counter.

### Evaluation of Inhibition of Cell to Cell Fusion in TZM-bl-FcRI Cells

HeLa-CD4-LTR-β-Gal cells are plated at a density of 5x10³ cells per well in a volume of 50 µL with 50 µL of six ½-log₁₀ serial dilutions of compound in triplicate for one hour at 37°C/5% CO₂. Following the incubation, 100 µL of HL2/3 cells are added to the plates. The cultures are incubated for an additional 48 hours at 37°C/5% CO₂. Following the incubation, efficacy plates are evaluated for β-galactosidase production using a chemiluminescent substrate and toxicity plates are stained with XTT to evaluate cell viability as described above.

### EVALUATION OF INHIBITION OF HIV-1 ENZYMES

### Evaluation of Inhibition of HIV-1 Reverse Transcriptase

The HIV-1 reverse transcriptase (RT) inhibition assay utilizes HIV-1 reverse transcriptase (RT) enzyme provided by ChimerX. Six concentrations serially diluted logarithmically in water were added to a 96-well U-bottom plate with 50 µL of a reaction mixture containing 2M Tris-HCl, pH 8.0, 3M KCl, 1M MgCl₂, 2M DTT, 10mM dGTP, 25U/mL rC:dG template, 10 µL [³²P]-α-dGTP (800 Ci/mMol) and 20 µL of enzyme reaction mix containing 5 µL of HIV-1 reverse transcriptase enzyme, BSA and Triton X-100. The reaction plate was incubated at 37° C for 50 minutes. Following the incubation, 10 µg/mL of sonicated salmon sperm DNA and 150 µL of 10% TCA was added to the wells to aid in the DNA precipitation and recovery and was allowed to incubate at room temperature for 15 minutes. The contents in the well were then transferred to a DEAE anion exchange paper and washed by suctioning through the filter using a vacuum manifold. The plate was then washed one time with 200 µL of 10% TCA as above. Fifteen microliters (15µL) of Wallac Supermix Scintillant was added to each well and the plate was read using a Wallac MicroBeta scintillation counter.

### Evaluation of Inhibition of HIV-1 Protease

HIV-1 protease activity is determined using the Sensolyte 520 HIV-1 Protease Assay Kit which incorporates a Hilyte Fluor^{™} 488/QXL^{™}520 FRET peptide. In the FRET peptide, the fluorescence of HiLyte Flour^{™}488 is quenched by QXL^{™}520 until this peptide is cleaved into two separate fragments by HIV-1 protease. Upon cleavage, the fluorescence of Hilyte Fluor^{™}488 is recovered and monitored at an excitation/emission = 490 nm /520 nm. Recombinant HIV-1 protease is diluted to a concentration of 2.5 ng/mL in protease assay buffer and 40 µL of the diluted protease is added to all but two wells of a NUNC 96-well flat bottom black fluorescence plate. Six ½-log₁₀ serial dilutions of test compounds and Saquinavir (positive control compound) are prepared to 10 times the final in well concentration in protease assay buffer. Ten microliters (10 µL) of the diluted compounds are placed into triplicate wells of the assay plate containing the protease. Ten microliters (10 µL) of buffer alone is also placed into six wells containing protease for establishing a no compound positive control. The plate is incubated at 37°C for 15 minutes. Fifty microliters (50 µL) of the fluorescent protease substrate (diluted 1:500 in assay buffer) is added to each well and the plate is incubated in room temperature for 60 minutes. Fifty microliters (50 µL) of stop solution is added to each well. Fluorescence intensity is measured using an excitation/emission=490 nm/520 nm. The EC₅₀ is calculated from the end-point fluorescence data for each of the compounds.

### Evaluation of Inhibition of HIV-1 Integrase

HIV-1 protease activity is evaluated using a HIV-1 Integrase Assay Kit (XpressBio Life Science). Streptavidin-coated 96-well plates are coated with a double-stranded HIV-1 LTR U5 donor substrate (DS) DNA containing an end-labeled biotin. One hundred microliters (100 µL) of 1X DS DNA solution is added to the designated wells and the plate incubated for 30 minutes at 37°C. Following the incubation, the plate is washed 3 times with wash buffer and 200 µL of blocking buffer is added to each well and incubated for 30 minutes at 37°C. One-hundred microliters (100 µL) of reaction buffer (negative control) or integrase enzyme solution (positive control) is added to the designated wells and incubated for 30 minutes at 37°C. The plate is washed as above and 50 µL of each test article diluted in reaction buffer (reaction buffer alone for positive and negative controls) is added to the designated wells and incubated for 5 minutes at room temperature. Fifty microliters (50 µL) of the 1X TS DNA solution is added the plate, mixed and the plate incubated for 30 minutes at 37°C. After washing the plate 5 times with wash buffer, 100 µL of HRP antibody solution is added and the plate incubated for 30 min at 37°C. Following an additional 5 washes, 100 µL of TMB peroxidase substrate solution is added and the plate incubated for 10 minutes at room temperature. One hundred microliters (100 µL) of TMB stop solution is directly added to the wells containing the TMB substrate. The plate is read at an absorbance at 450 nM.

### Evaluation of Time of Compound Addition in HeLa-CD4-LTR-β-Gal Cells

HeLa-CD4-LTR-β-Gal cells are seeded at a density of 1x10⁴ cells/well in a volume of 100 µL 24 hours prior to assay initiation and incubated at 37°C/5% CO₂. Following the incubation compound is serially diluted at the specified concentrations and were added to the cells in triplicate at timepoints of -30 minutes, 0, 1, 2, 4, 8 and 24 hours pre- or post- virus addition in a volume of 100 µL. HIV-1_{IIIB} is added to the cells at a pre-determined titer. The cultures are incubated at 37°C/5% CO₂ for 48 hours at which time efficacy is evaluated using a chemiluminescent substrate (Gal-Screen) and toxicity (three timepoints of 0, 4 and 24 hours) evaluated using the tetrazolium dye XTT.

### Evaluation of Activity Against HBV in AD38 Cells:

AD38 cells contain a stably transfected HBV genome under the transcriptional control of the tet operon. Expression of HBV is repressed when the cells are cultured in the presence of tetracycline and can be induced with the removal of tetracycline from the culture medium. AD38 cells are seeded in a 96-well flat bottomed plate at a density of 1x10⁵ cells per well and are cultured in the presence of 0.3 µg/ml tetracycline for 2 days at 37°C/5% CO₂. Following the incubation, the media is removed and the cells washed to remove residual tetracycline. Six concentrations of serially diluted polymer (1/2 log increments) are added to the cells and incubated for 6 days at 37°C/5% CO₂ changing the media on day 3 (polymer added back). On the sixth day, 100 µL of supernatant is collected from each well for analysis of viral DNA by qPCR and the cell monolayers re stained with XTT to evaluate cytotoxicity as described above.

### qPCR Methodology:

One-hundred microliters (100 µL) of cell culture supernatant is diluted with 90 µL of dilution buffer containing 10 µM Tris and 40 µg/ml of Salmon DNA. The samples are heated to 102°C for 15 minutes. Six microliters (6 µL) of sample is then mixed with 12.5 µL of 2X Platinum PCR super mix with ROX, 0.5 µL of the HBV forward primer (10 µM AD38 qF1),0.5 µL of the HBV reverse primer (10 µM AD38qR1), 0.5 µL of the Taqman Probe (AD38 qP1) and 6 µL of molecular grade water. Polymerase Chain Reaction (PCR) is performed under the following conditions: 50 cycles of 95°C for 15 seconds followed by 60°C for 1 minute.

### Evaluation of Activity Against Influenza A and Influenza B Virus:

MDCK cells cultured in DMEM supplemented with 10% FBS, 2 mM L-glutamine, 100 U/ml penicillin, 100 µg/ml streptomycin 1 mM sodium pyruvate, and 0.1 mM NEAA are seeded in a 96-well flat-bottomed plate at a cell density of 1x10⁴ cells per well in a volume of 100 µL. The plates are incubated at 37°C/5% CO₂ for 24 hours. Following the incubation, the polymers are serially diluted in half logarithmic increments (6 concentrations total) and 100 µL of each concentration is added to the cells in triplicate. Influenza A_{CA/27/07} or Influenza B_{Allen} virus is diluted to a predetermined titer in assay medium and added to the cultures in a volume of 100 µL. This titer of virus is the amount determined to yield 80% cell killing at 4 days post-infection. The cultures are incubated for 4 days at 37°C/5% CO₂. Following the incubation the test plates are stained with the tetrazolium dye XTT as described above.

### Evaluation of Activity Against Adenovirus and Rhinovirus:

Inhibition of virus-induced cytopathic effects (CPE) and cell viability following adenovirus replication in HeLa cells or human rhinovirus replication in MRC-5 cells is measured by XTT tetrazolium dye. Cells (1 x 10⁴ cells per well) are seeded in 96-well flat-bottom tissue culture plates and allowed to adhere overnight at 37°C/5% CO₂. Following incubation, media is removed from the cell monolayers and serially diluted polymer (6 concentrations) and virus diluted to a pre-determined titer to yield 85 to 95% cell killing at 6 days post-infection are added to the plate. Ribavirin is evaluated in parallel as a positive assay control compound. Following incubation at 37°C, 5% CO₂ for six days, cell viability is measured by XTT staining as described below.

### Evaluation of Toxicity to Ca Ski and ME180 Cells

Twenty-four hours prior to compound exposure, 100 µL of cells are plated at 5x10⁴ cells per well in a flat-bottomed plate and. Following a 24 hour incubation at 5%CO₂/37°C serially diluted compound and media is added to the cells in triplicate. The cultures are incubated for an additional 24 hours at 5%CO₂/37°C, at which time they are washed to remove residual compound. The plates are incubated at 5% CO₂/37°C for an additional 24 hours and then evaluated using XTT as described above.

### Evaluation of Toxicity to the Normal Vaginal Flora Lactobacillus Species

A frozen glyercol stock of *Lactobacillus jensenii* and *Lactobacillus crispatus* are grown in MRS broth for 48 hours prior to exposure of compounds. Six (6) concentrations of each compound are serially diluted in MRS broth and each was added in triplicate to a 96-well round bottom plate. Each species of *Lactobacillus* is diluted to an OD₆₂₅ = 0.06 in MRS broth and added to the appropriate wells of the plate. The cultures are incubated anaerobically for 24 hours at which time bacterial growth is evaluated spectrophotometrically at 490 nM. Penicillin/Streptomycin solution is used as an assay control.

### Evaluation of Toxicity to Vaginal Ectocervical Tissue

On the day of the assay, the MatTek assay media is warmed and 900 µL added to each well of a 6-well plate. One hour prior to dosing, the epivaginal tissues are removed from the refrigerator and placed in the 6-well plate and the plate then incubated for 1 hour at 37°C/5% CO₂. Following the incubation the media is removed from the 6 well plate and 900 µL of fresh media added. One-hundred microliters (100 µL) of each concentration is added in duplicate to the epivaginal tissue. The plate is then incubated for an additional 24 hours at 37°C/5% CO_{2.} One hour prior to the end of the incubation, 1 mg/mL (3-(4,5-Dimethylthiazol-2-yl)-2,5-diphenyltetrazolium bromide (hereinafter, "MTT" available from Invitrogen of Carlsbad, California) in DMEM is prepared. Fifteen minutes prior to the end of the incubation 300 µL of MTT is added to each well of a 24-well plate. Following the incubation, residual liquid is removed from the tissue and then gently rinsed two times with PBS. Each epivaginal tissue insert is then transferred to an individual well of the 24-well plate containing the MTT solution. The plate is then incubated for 3 hours at 37°C/5% CO₂. Each insert is then transferred to a pre-labeled extraction plate and 2.0 mL of extracting solution added to the well so that the tissue insert was fully submerged. The extraction proceeded for two hours at room temperature at which time the extracting solution is mixed thoroughly and 200 µL is transferred to a 96-well round bottom plate. The optical density of each sample is determined at 570 nm with a background of 650 nm. The % viability is determined to be 100 x [OD(sample)/OD(negative control)]. Triton X-100 and N-9 are used as assay controls.

### Materials:

A low molecular weight hydrophobically modified polymer, Potassium Acrylates Copolymer (Lubrizol, Brecksville, OH) was used in the compositions of this invention as the low molecular weight hydrophobically modified polymer.

### Example 1

### Inventive examples E1 - E3 and Comparative examples C1-C3: Preparation of Compositions to be tested

The compositions of E1-E3, A1 and C1-C3 were prepared according to the descriptions set forth below with materials in the amounts listed in Table 1. Compositions E1-E3 are in accordance with the compositions and methods of this invention. Composition A1 is in accordance with compositions and methods set forth in co-pending patent application Attorney Docket No.JCO6079USNP filed concurrently herewith. Compositions C1-C3 are comparative compositions.

**Table 1**

| **Ingredient** | **E1** | **A1** | **C1** | **C2** | **E2** | **E3** | **C3** |
|---|---|---|---|---|---|---|---|
| **INCI name** | **w/w %** | **w/w %** | **w/w %** | **w/w %** | **w/w %** | **w/w %** | **w/w %** |
| Potassium Acrylates Copolymer | 0.50 | - | - | 0.50 | 3.0 | 3.0 | - |
| PEG6000 | - | 0.5 | - | - | - | - | - |
| Cocamidopropyl Betaine | - | - | 3.0 | 3.0 | - | - | - |
| Sodium laureth Sulfate | - | - | 3.0 | 3.0 | - | - | - |
| PEG 80 Sorbitan Laurate | - | - | 3.0 | 3.0 | - | - | - |
| Nonoxynol-9 | - | - | - | - | - | 3.0 | 3.0 |
| Sodium Hydroxide | qs | qs | qs | qs | qs | qs | qs |
| Water | qs | qs | qs | qs | qs | qs | qs |

| | | | | | | | |
|---|---|---|---|---|---|---|---|
| *expressed in %w/w actives | | | | | | | |

Each of the compositions of Table 1 was independently prepared as follows:
E1 - 1.7 g of Potassium Acrylates Coploymer (Activity 30 %) was mixed with 98.3 g of deionized water and the pH adjusted to 6.5 using 20 % Sodium Hydroxide solution.
A1-0.5 g of PEG6000 was dissolved in water with slight heating and the pH measured was 6.65.
C1- 17.4 g of Cocamidopropyl Betaine, 23.4 g of Sodium laureth Sulfate and 8.3 gm of PEG 80 Sorbitan Laurate were added to 150.9 g of deionized water and the pH was measured at 6.8.
C2 - 3.4 g of Potassium Acrylates Copolymer, 17.4 g of Cocamidopropyl Betaine, 23.4 gm of Sodium laureth Sulfate and 8.3 g of PEG 80 Sorbitan Laurate were added to 150.9 gm of deionized water and the Potassium Acrylates Copolymer was neutralized using 20 % Sodium Hydroxide. The final pH was measured was 6.5.
E2 - 10.0 gm of Potassium Acrylates Copolymer (Activity 30 %) was mixed with 88.56 gm of deionized water and the pH adjusted to 6.7 using 1.44 gm of 20 % Sodium Hydroxide solution.
E3 - 3 gm of Nonoxynol-9 was mixed with 86.24 gm of deionized water and stirred on the mixing plate until the Nonoxynol-9 completely dissolved. 10 gm of Potassium Acrylates Copolymer was added to the mixture and the pH adjusted to 6.4 - 6.6 using 20 % Sodium Hydroxide solution.
C3 - 3 gm of Nonoxynol 9 was mixed with 97 gm of deionized water and stirred on a mixing plate till Nonoxynol completely dissolved and the pH measured was 5.2-5.4.

### Example 2

### Inventive Examples E4 - E11: Preparation of Illustrative Embodiments of the Compositions of This Invention

Stable anti-viral compositions of E4 - E11 were prepared according to the materials and amounts listed in Table 2 and the methods set forth below.

**Table 2**

| | **E4** | **E5** | **E6** | **E7** | **E8** | **E9** | **E10** | **E11** |
|---|---|---|---|---|---|---|---|---|
| **INCI name** | **w/ w %** | **w/w %** | **w/w %** | **w/w %** | **w/w %** | **w/w %** | **w/w %** | **w/w %** |
| Carbomer | 0.7 5 | 0.75 | - | - | 0.75 | - | - | - |
| Potassium Cetyl Phosphate (and) Hydrogenated Palm Glycerides | 2.0 | 2.0 | - | - | 2.0 | - | - | - |
| Stearyl Alcohol | | 0.37 5 | | | 0.37 5 | - | - | - |
| Polyglyceryl-10 Laurate | | 0.37 5 | | | | | | |
| Phenoxyethanol; Ethylhexylglycerine | 2.5 | 2.5 | - | 0.6 | 2.5 | - | 0.6 | - |
| Mineral Oil | 8.0 | 8.0 | - | - | 8.0 | - | - | - |
| Propylene Glycol | - | - | - | - | - | 39.9 4 | | |
| Potassium Acrylates Copolymer | 5 | 10 | 5.0 | 0.5 | 0.05 | 0.05 | 0.0 5 | 0.0 5 |
| Hydroxyethylcellulose | - | - | - | 0.2 | - | 1.6 | 0. 2 - | - |
| Carbomer | - | - | 15 | - | - | - | - | 15 |
| Methylchloroisothi azolinone(and) Methyl isothiazolinone | - | - | 0.2 | - | - | - | - | 0.2 |
| Coco-Glucoside (and)Glyceryloleate | - | - | - | 0.2 5 | - | - | - | - |
| Lauryl Glucoside (and) Polyglyceryl-2 Dipoly-hydroxystearate (and) Glycerin | - | - | - | 0.2 5 | - | - | - | - |
| Glycerin | - | - | - | 0.1 8 | - | - | 0.1 8 | - |
| Sodium Benzoate | - | - | - | 0.5 | - | 0.2 | 0.5 | - |
| Acrylates Crosspolymer | - | - | - | 0.5 | - | - | 0.5 | - |
| Sodium Hydroxide | qs | qs | qs | qs | qs | qs | qs | qs |
| Water | qs | qs | qs | qs | qs | qs | qs | qs |

| | | | | | | | | |
|---|---|---|---|---|---|---|---|---|
| *expressed in %w/w actives | | | | | | | | |

Each of the embodiment compositions of Table 2 was independently prepared as follows:
**E4-E5 - For E4:** Water was measured in the main beaker. Carbomer was dusted into the water while mixing. Carbomer was permitted to disperse uniformly. Potassium Acrylates Copolymer was added and heating started until the temperature reached 65° C. Potassium Cetyl Phosphate (and) Hydrogenated Palm Glycerides, Mineral Oil were added to the mixture. After 10 minutes, the heat was turned off and the mixture cooled. At room temperature, Phenoxyethanol;ethylhexylglcerine was added and the composition's pH adjusted to 6.7-7.00 qs and store. The pH of the lotion was recorded as 6.8.
For **E5,** the same procedure was followed as E5 with the exception that Polyglyceryl-10 Laurate and Stearyl Alcohol was added after the mineral oil was added to the mixture.
**E6** - Acrylates Crosspolymer and water were added together into a beaker. Potassium Acrylates Copolymer was added and neutralized using 20 % Sodium hydroxide solution. A clear gel was formed. The pH was recorded as 7.2.
**E7** - Water was added to a beaker and Hydroxyethylcellulose stirred into the water and allowed to mix for 45 min. The mixture was heated to 50° C. Potassium Acrylates Copolymer, Coco-Glucoside (and) Glyceryl Oleate, Lauryl Glucoside (and) Polyglyceryl-2 Depolyhydroxystearate (and) Glycerin were added to the beaker. The heat was turned off and, when the composition reached about 30°C, Glycerin, Phenoxyethanol;ethylhexylglcerine and Sodium Benzoate were added to the beaker. The pH was adjusted to 7 and Acrylates Crosspolymer was added.
**E8** - Water was added to a beaker and mixed. Carbomer was dusted in while mixing. The Carbomer was permitted to disperse uniformly. Potassium Acrylates Copolymer was added to the composition and heating initiated until the temperature reached 65° C. Potassium Cetyl Phosphate(and) Hydrogenated Palm Glycerides, Stearyl Alcohol and Mineral Oil were added to the composition. The heat was turned off after 10 min and the mixture permitted to cool to room temperature. Phenoxyethanol; ethylhexylglcerine was added and the pH adjusted to 6.7-7.00, qs and stored.
**E9** - Water and Propylene Glycol were added to a main beaker. Potassium Acrylates Copolymer was added to the beaker with agitation. Sodium Hydroxide was added to neutralize Potassium Acrylates Copolymer (until the solution turned clear and pH was between 6.5 and 7). Sodium Benzoate was added and the composition permitted to mix for 30 minutes. The mixture was heated to 55 to 60° C. Hydroxyethylcellulose was added slowly and the composition mixed until a smooth gel was obtained. The mixture was cooled to room temperature and the pH of the gel recorded at 4.5.
**E10** - To a beaker add water, Carbomer while mixing. Stir in the Hydroxyethylcellulose and let mix for 45 minutes while heating up to 50° C. Remove from heat and when the composition reaches about 30° C, add Potassium Acrylates Copolymer. Add Sodium Hydroxide to neutralize Potassium Acrylates Copolymer (till the solution turns clear and pH is between about 6.5 and about 7. Add Glycerin, Phenoxyethanol; ethylhexylglcerine and Sodium Benzoate. qs and adjusted pH to 6.5-7.0.
**E11** - Carbomer and water were added together to a beaker. Kathon CG was then added. Subsequently, Potassium Acrylates Copolymer was added to the composition and neutralized using 20 % Sodium Hydroxide solution with agitation. A clear gel was formed and the pH recorded at 7.2.

### Example 3

### Mildness testing via Trans Epithelium Permeation (TEP) Test

Samples of E2, E3 and C3 were tested for TEP as per the method detailed set forth above.

**Table 3**

| Sample | TEP: EC₅₀ |
|---|---|
| E2 | >8 |
| E3 | 4.77 +/- 0.77 |
| C3 | 3.46 +/- 0.62 |

C3, which contains Nonoxynol N9 and water, demonstrated significant leakage compared to E2 and E3, both of which contained Potassium Acrylates Copolymer (E3 also containing Nonoxynol N9). Both E2 and E3 demonstrated superior mildness in TEP assay whereas C3 shows potential for membrane-damage that may result in penetration of agents via mucosal tissues.

### Example 4

### Virucidal effect of Potassium Acrylates Copolymer (hydrophobically modified polymer) using an in-vitro time-kill method against HSV-1.

Using the protocol described above, neutralization studies of E1 were performed versus the challenge virus strain to ensure that the neutralizing solution employed (De Engle [D/E] Neutralizing Broth) was effective in neutralizing the virucidal activity of the product. The neutralizing solution (D/E) effectively neutralized the virucidal activity of the test product and was shown to be non-toxic to the virus and cell cultures.

**Table 4**

| Dilution (- Log₁₀) | Virus Control | Test Exposure Time | | | Cytotoxicit y Control | Neutraliza -tion Control | Cell Control |
|---|---|---|---|---|---|---|---|
| | | 15 minutes | 30 minutes | 60 minutes | | | |
| | | | | | | | 0000 |
| -2 | NT | CT | CT | CT | CT | NT | |
| -3 | ++++ | 0000 | 0000 | 0000 | 0000 | ++++ | |
| -4 | ++++ | 0000 | 0000 | 0000 | 0000 | ++++ | |
| -5 | ++++ | 0000 | 0000 | 0000 | NT | ++++ | |
| -6 | +++0 | 0000 | 0000 | 0000 | NT | +00+ | |
| -7 | 00+0 | 0000 | 0000 | 0000 | NT | 0000 | |
| TCID₅₀ log₁₀ | 6.50 | 2.50 | 2.50 | 2.50 | 2.50 | 6.00 | |
| Log₁₀ Reducti on | N/A | 4.00 | 4.00 | 4.00 | N/A | | |
| Percent Reducti on | N/A | 99.99 % | 99.99% | 99.99% | | | |

| | | | | | | | |
|---|---|---|---|---|---|---|---|
| + CPE (cytopathic/cytotoxic effect) present 0 CPE (cytopathic/cytotoxic effect) not detected NT Not tested N/A Not applicable CT Cytotoxicity | | | | | | | |

The virus population recovered from Virus Control #1 was 6.50 log₁₀, from Virus Control#2 was 6.25 log₁₀, and from the Neutralization Control was 6.25 log₁₀. The observed differences did not exceed 1 log₁₀, thus the test virus infectivity was not affected.

Surprisingly, the E1 composition containing Potassium Acrylates Copolymer reduced the infectivity of the HSV-1 strain HF (ATCC#VR-260) by 4.00 log₁₀ (99.99%) following 15-minute, 30-minutes, and 1 hour exposure (Table 4).

### Example 5

### Activity of Embodiments against HIV-1, Hepatitis B, Influenza, Adenovirus, and Rhinovirus

Following the protocol described above, embodiments E1, A1, C1 and C2 were tested against a broad range of HIV-1 subtypes and tropisms representing the breadth of HIV-1 global diversity (Table 5).

**Table 5**

| **Virus** | **Strain** | **Tropis m/Subt ype** | **E1** | **A1** | **C1** | **C2** |
|---|---|---|---|---|---|---|
| | | | **EC50 (µg/ml)** | **EC50 (µg/ml)** | **EC50 (µg/ml)** | **EC50 (µg/ml)** |
| HIV-1 | IIIB | CXCR4/ B | 0.18 | >2500* | >2.50* | >2.50* |
| HIV-1 | 92RW0 16 | CCR5/ A | 5.48 | nd | nd | nd |
| HIV-1 | 91US05 6 | CCR5/ B | 1.52 | 179.7* | 2.96* | 5.10* |
| HIV-1 | 92BR01 4 | CXCR4/ B | 1.86 | nd | nd | nd |
| HIV-1 | 97ZA00 9 | CCR5/ C | 3.82 | nd | nd | nd |
| HIV-1 | 92UG00 1 | CXCR4/ CCR5/D | 2.15 | nd | nd | nd |
| HIV-1 | CMU02 | CXCR4/ E | 7.12 | nd | nd | nd |
| HIV-1 | 93BR02 0 | CXCR4/ CCR5/F | 5.45 | nd | nd | nd |
| HIV-1 | G3 | CCR5/ G | 4.93 | nd | nd | nd |
| HIV-1 | BCF01 | CCR5/ O | 15.5 | nd | nd | Nd |
| Influenza | A_{CA/27/07} | n/a | >2500* | 50 | >25* | >25* |
| Influenza | BAllen | n/a | >2500* | 20 | >25* | >25* |
| HBV | n/a | n/a | 45.2* | >2500 | 29.3* | 15.2* |
| HBV | n/a | n/a | 17.29* | nd | nd | nd |
| Adenovir us | n/a | n/a | >5 | nd | nd | nd |
| Rhinovir us | n/a | n/a | >5 | nd | nd | nd |

| | | | | | | |
|---|---|---|---|---|---|---|
| * Formulas are toxic to cells at levels at or below inhibitory concentrations. No activity determined. nd: not done | | | | | | |

Embodiment E1 also showed similar activity against several other HIV-1 strains representing further geographic and genetic diversity, including 92UG029, 92HT599, 98IN017, 92UG024, 92RW020, 92BR003, 97ZA003, 92UG035, and 93TH073, but not against influenza. Embodiment A1 surprisingly showed activity against influenza virus, while not showing activity against other enveloped viruses tested, as reflected in copending patent application Attorney Docket No. JCO6079USNP. We theorize that compositions containing polyalkylene glycols would be effective in inhibiting influenza viruses, more preferably polyethylene glycols and polypropylene glycols, most preferably polyethylene glycols having higher molecular weight, such as about 6000. We further theorize that compositions containing both low molecular weight hydrophobically modified polymers and polyalkylene glycols should have broad spectrum activity in inhibiting enveloped viruses including influenza.

As shown, the inventive embodiments demonstrate activity at very low concentrations against a surprisingly broad range of HIV-1 subtypes and tropisms. These data demonstrate the broad spectrum activity of embodiments E1 against HIV-1.

### Example 6: Mechanism of anti-HIV-1 Activity

Embodiment E1 was evaluated in multiple *in vitro* HIV-1 mechanism of action determination assays to evaluate and define its anti-HIV mechanism of action.

### Results:

As shown in Table 6, E1 had no effect on treating cells chronically infected with HIV-1. Additionally, Embodiment E1 was inactive in preventing cell to cell transmission of HIV-1 or preventing the fusion of HIV-1 infected cells. Embodiment E1 did show efficacy in the entry inhibition assays in TZM-bl-FcRI cells, suggesting that the polymer is a weak entry inhibitor. E1 was able to inhibit the HIV-1 enzymes of integrase, protease, and reverse transciptase; however, this activity is likely non-specific and is not expected to contribute to its efficacy against virus in cell culture. Based on the data contained in Tables 6 and 7, Embodiment E1 likely blocks an early step in HIV replication, such as virus attachment, fusion, or entry into target cells, but prior to reverse transcription. The polymer must be present within the first 1-2 hours following infection, consistent with other actives that block the early steps in viral replication.

**Table 6:**

| Assay | Virus | Cell Type | E1 | |
|---|---|---|---|---|
| | | | EC50 | TI |
| Chronic Virus Replication Inhibition | HIV-1_{IIIB} | CEM-SS | 9.1 | <1 |
| | | | 9,6 | <1 |
| Chronic Cell to Cell Transmission Inhibition | HIV-1_{IIIB} | CEM-SS | >10 | - |
| | | | 1 | - |
| Entry Inhibition | HIV-1_{BaL} | TZM-bl FcRI (Hela) | 21.01 | >11.9 |
| Fusion Inhibition | | HeLa-CD4-LTR-B-Gal | 38.6 | 1 |
| Reverse Transcriptase Inhibition | n/a | n/a | 31.43 | - |
| Protease Inhibition | n/a | n/a | 0.48 | - |
| Integrase Inhibition | n/a | n/a | 0.74 | - |

| | | | | |
|---|---|---|---|---|
| TI: therapeutic index (TC50/EC50) *activity artifact due to E1 polymeric nature | | | | |

**Table 7:**

| **Compound (MOA)** | **Time in which Antiviral Activity is Lost** |
|---|---|
| Efavirenz (Reverse Transcriptase) | 4-8 hours |
| T20 (Fusion) | 0-2 hours |
| PRO2000 (CD4/gp120 attachment) | 0-2 hours |
| E1 | 0-2 hours |

### Example 7 - Mucosal Cellular Toxicity Testing

Example E1 was tested for mucosal cellular toxicity using the protocol described above. E1 was compared to the broadly active, antiviral surfactant N-9. In all tested cases, E1 was less toxic to cells and tissue than Nonoxynol-9, as set forth in Table 8 Additionally, toxic concentration of E1 were 100-1000 times the concentrations of E1 that showed efficacy against HIV-1 (Table 7). These data demonstrate the mildness of E1 and similar embodiments on mucosal tissue.

**Table 8**

| Cells | Exposure | E1 | N-9 µg/ml |
|---|---|---|---|
| Ca Ski (Cervical) | 24h | 109.11 | 36.31 |
| ME180 (Cervical) | 24h | 133.59 | 41.83 |
| Vaginal Ectocervical Tissues | 24h | >5000 | 100-1000 |

Embodiment E1 also demonstrated to have no toxicity on *Lactobacillus* populations at biologically relevant concentrations (TC50 >1450µg/ml) in the testing described above. This further demonstrates its compatibility with mucosal surfaces.

## Claims

1. An anti-viral composition for use in a method of inhibiting entry of enveloped viruses into cells,
wherein said method comprises contacting said viruses with said anti-viral composition comprising at least one low molecular weight hydrophobically modified polymer in an amount effective to inhibit entry of viruses into cells,
wherein said method comprises applying said anti-viral composition to infectable surfaces of a subject, said infectable surfaces comprising one or more of the group consisting of skin and mucosal tissue of a subject,
wherein said low molecular weight hydrophobically modified polymer comprises a polymer derived from at least one first monomeric component selected from the group consisting of (meth)acrylic acid and at least one second monomeric component selected from the group consisting of one or more C₁ to C₉ alkyl (meth)acrylates,
wherein the low molecular weight copolymer has a number average molecular weight of 100,000 Daltons or less, and
wherein said composition is substantially free of anionic, cationic or amphoteric surfactant having a hydrophilic-lipophilic balance (HLB) of greater than 12.

2. The anti-viral composition for use according to claim 1 wherein said mucosal tissue comprises tissue selected from the group consisting of oral tissue, ocular tissue, nasal tissue, vaginal tissue, or rectal tissue and a combination thereof.

3. The anti-viral composition for use according to claim 1 wherein said low molecular weight hydrophobically modified polymer is present in said composition in an amount of from 0.00005% to 10% by weight of the composition.

4. The anti-viral composition for use according to claim 1 wherein said composition further comprises at least 50% of protic solvent.

5. The anti-viral composition for use according to claim 4 wherein said composition comprises at least 97% of water.

6. The anti-viral composition for use according to claim 1 wherein said viruses are selected from the group consisting of poxviridae, herpesviridae, retroviridae Lentivirus and a combination thereof.

7. The anti-viral composition for use according to claim 6 wherein:
(a) said virus selected from the family of herpesviridae is herpes simplex virus 1, herpes simplex virus 2 and a combination thereof; or
(b) said virus selected from the family retroviridae Lentivirus is Human Immunodeficiency Virus Type 1.

8. The anti-viral composition for use according to claim 1 wherein:
(a) said inhibition of virus entry into said cells results in the reduction of potential for viral infection; or
(b) the anti-viral composition does not substantially disrupt biological surfaces.

9. A method of inhibiting the transmission of viruses,
comprising applying to non-biological surfaces a composition comprising at least one low molecular weight hydrophobically modified polymers in an amount effective to inhibit entry of viruses into cells,
wherein said composition is substantially free of anionic, cationic or amphoteric surfactant,
wherein said low molecular weight hydrophobically modified polymer comprises a polymer derived from at least one first monomeric component selected from the group consisting of (meth)acrylic acid and at least one second monomeric component selected from the group consisting of one or more C₁ to C₉ alkyl (meth)acrylates, and
wherein the low molecular weight copolymer has a number average molecular weight of 100,000 Daltons or less.

10. A method of inhibiting the transmission of viruses,
comprising applying to ingestable surfaces a composition comprising at least one low molecular weight hydrophobically modified polymers in an amount effective to inhibit entry of viruses into cells,
wherein said composition is substantially free of anionic, cationic or amphoteric surfactant,
wherein said low molecular weight hydrophobically modified polymer comprises a polymer derived from at least one first monomeric component selected from the group consisting of (meth)acrylic acid and at least one second monomeric component selected from the group consisting of one or more C₁ to C₉ alkyl (meth)acrylates, and
wherein the low molecular weight copolymer has a number average molecular weight of 100,000 Daltons or less.

11. The anti-viral composition for use according to any one of claims 1 to 8, or the method according to claim 9 or claim 10, wherein said composition comprises a dosage form selected from the group consisting of: a liquid, a lotion, a cream, a gel, a spray, a shaving cream, an ointment, a cleansing liquid wash, a shampoo, a paste, a mousse, a patch, and a hydrogel.

12. The anti-viral composition for use according to any one of claims 1 to 8 or claim 11, or the method according to any one of claims 9 to 11, wherein said hydrophobically modified low molecular weight polymer comprises a low molecular weight, non-crosslinked, linear acrylic copolymer derived from at least one first monomeric component selected from the group consisting of (meth)acrylic acid and at least one second monomeric component selected from the group consisting of one or more C₁ to C₉ alkyl (meth)acrylates, wherein the low molecular weight copolymer has a number average molecular weight of 100,000 Daltons or less.

13. The anti-viral composition for use according to any one of claims 1 to 8, claim 11 or claim 12, or the method according to any one of claims 9 to 12, wherein said hydrophobically modified low molecular weight polymer is potassium acrylates copolymer.

14. The anti-viral composition for use or the method according to claim 13 wherein said composition further comprises nonoxynol-9.

## Patentansprüche

1. Antivirale Zusammensetzung zur Verwendung bei einem Verfahren zum Hemmen des Eindringens umhüllter Viren in Zellen,
wobei das Verfahren das Inkontaktbringen der Viren mit der antiviralen Zusammensetzung umfasst, die mindestens ein niedermolekulares, hydrophob modifiziertes Polymer in einer Menge umfasst, die eine Hemmung des Eindringens von Viren in Zellen bewirkt,
wobei das Verfahren das Aufbringen der antiviralen Zusammensetzung auf infizierbare Oberflächen eines Individuums umfasst, wobei die infizierbaren Oberflächen eine oder mehrere der aus Haut- und Schleimhautgewebe eines Individuums bestehenden Gruppe umfassen,
wobei das niedermolekulare, hydrophob modifizierte Polymer ein Polymer umfasst, das von mindestens einer ersten monomeren Komponente, die aus der aus (Meth)Acrylsäure bestehenden Gruppe ausgewählt ist, und mindestens einer zweiten monomeren Komponente, die aus der aus einem oder mehreren C₁- bis C₉-Alkyl(meth)acrylaten bestehenden Gruppe ausgewählt ist, stammt,
wobei das niedermolekulare Copolymer ein zahlenmittleres Molekulargewicht von 100.000 Dalton oder weniger aufweist und
wobei die Zusammensetzung im Wesentlichen frei von anionischem, kationischem oder amphoterem Tensid mit einem Hydrophil-Lipophil-Gleichgewicht (HLB) von mehr als 12 ist.

2. Antivirale Zusammensetzung zur Verwendung nach Anspruch 1, wobei das Schleimhautgewebe Gewebe umfasst, das aus der aus Mundgewebe, Augengewebe, Nasengewebe, Vaginalgewebe oder Rektalgewebe und einer Kombination davon bestehenden Gruppe ausgewählt ist.

3. Antivirale Zusammensetzung zur Verwendung nach Anspruch 1, wobei das niedermolekulare, hydrophob modifizierte Polymer in der Zusammensetzung in einer Menge von 0,00005 Gew.- % bis 10 Gew.- %, bezogen auf das Gewicht der Zusammensetzung, vorliegt.

4. Antivirale Zusammensetzung zur Verwendung nach Anspruch 1 , wobei die Zusammensetzung ferner mindestens 50 % protisches Lösungsmittel umfasst.

5. Antivirale Zusammensetzung zur Verwendung nach Anspruch 4, wobei die Zusammensetzung mindestens 97 % Wasser umfasst.

6. Antivirale Zusammensetzung zur Verwendung nach Anspruch 1, wobei die Viren aus der aus Poxviridae, Herpesviridae, Retroviridae Lentivirus und einer Kombination davon bestehenden Gruppe ausgewählt sind.

7. Antivirale Zusammensetzung zur Verwendung nach Anspruch 6, wobei:
(a) das aus der Familie der Herpesviridae ausgewählte Virus Herpes simplex-Virus 1, Herpes simplex-Virus 2 und eine Kombination davon ist oder
(b) das aus der Familie Retroviridae Lentivirus ausgewählte Virus Humanes Immundefizienzvirus Typ 1 ist.

8. Antivirale Zusammensetzung zur Verwendung nach Anspruch 1, wobei:
(a) die Hemmung des Viruseindringens in die Zellen zu einer Verringerung des Potenzials einer Virusinfektion führt oder
(b) die antivirale Zusammensetzung biologische Oberflächen nicht wesentlich beeinträchtigt.

9. Verfahren zur Hemmung der Übertragung von Viren,
umfassend das Aufbringen einer Zusammensetzung, die mindestens ein niedermolekulares, hydrophob modifiziertes Polymer in einer Menge umfasst, die eine Hemmung des Eindringens von Viren in Zellen bewirkt, auf nicht-biologische Oberflächen,
wobei die Zusammensetzung im Wesentlichen frei von anionischem, kationischem oder amphoterem Tensid ist,
wobei das niedermolekulare, hydrophob modifizierte Polymer ein Polymer umfasst, das von mindestens einer ersten monomeren Komponente, die aus der aus (Meth)Acrylsäure bestehenden Gruppe ausgewählt ist, und mindestens einer zweiten monomeren Komponente, die aus der aus einem oder mehreren C₁- bis C₉-Alkyl(meth)acrylaten bestehenden Gruppe ausgewählt ist, stammt, und
wobei das niedermolekulare Copolymer ein zahlenmittleres Molekulargewicht von 100.000 Dalton oder weniger aufweist.

10. Verfahren zur Hemmung der Übertragung von Viren,
umfassend das Aufbringen einer Zusammensetzung, die mindestens ein niedermolekulares, hydrophob modifiziertes Polymer in einer Menge umfasst, die eine Hemmung des Eindringens von Viren in Zellen bewirkt, auf ingestierbare Oberflächen,
wobei die Zusammensetzung im Wesentlichen frei von anionischem, kationischem oder amphoterem Tensid ist,
wobei das niedermolekulare, hydrophob modifizierte Polymer ein Polymer umfasst, das von mindestens einer ersten monomeren Komponente, die aus der aus (Meth)Acrylsäure bestehenden Gruppe ausgewählt ist, und mindestens einer zweiten monomeren Komponente, die aus der aus einem oder mehreren C₁- bis C₉-Alkyl(meth)acrylaten bestehenden Gruppe ausgewählt ist, stammt, und
wobei das niedermolekulare Copolymer ein zahlenmittleres Molekulargewicht von 100.000 Dalton oder weniger aufweist.

11. Antivirale Zusammensetzung zur Verwendung nach einem der Ansprüche 1 bis 8 oder Verfahren nach Anspruch 9 oder Anspruch 10, wobei die Zusammensetzung eine Dosierungsform umfasst, die aus der aus: einer Flüssigkeit, einer Lotion, einer Creme, einem Gel, einem Spray, einer Rasiercreme, einer Salbe, einer Reinigungswaschflüssigkeit, einem Shampoo, einer Paste, einem Schaum, einem Pflaster und einem Hydrogel bestehenden Gruppe ausgewählt ist.

12. Antivirale Zusammensetzung zur Verwendung nach einem der Ansprüche 1 bis 8 oder Anspruch 11 oder Verfahren nach einem der Ansprüche 9 bis 11, wobei das hydrophob modifizierte, niedermolekulare Polymer ein niedermolekulares, nicht vernetztes, gerades AcrylCopolymer umfasst, das von mindestens einer ersten monomeren Komponente, die aus der aus (Meth)Acrylsäure bestehenden Gruppe ausgewählt ist, und mindestens einer zweiten monomeren Komponente, die aus der aus einem oder mehreren C₁- bis C₉-Alkyl(meth)acrylaten bestehenden Gruppe ausgewählt ist, umfasst, wobei das niedermolekulare Copolymer ein zahlenmittleres Molekulargewicht von 100.000 Dalton oder weniger aufweist.

13. Antivirale Zusammensetzung zur Verwendung nach einem der Ansprüche 1 bis 8, Anspruch 11 oder Anspruch 12 oder Verfahren nach einem der Ansprüche 9 bis 12, wobei das hydrophob modifizierte, niedermolekulare Polymer Kaliumacrylate-Copolymer ist.

14. Antivirale Zusammensetzung zur Verwendung oder Verfahren nach Anspruch 13, wobei die Zusammensetzung ferner Nonoxynol-9 umfasst.

## Revendications

1. Composition antivirale pour une utilisation dans un procédé d'inhibition de l'entrée de virus enveloppés dans des cellules,
dans laquelle ledit procédé comprend la mise en contact desdits virus avec ladite composition antivirale comprenant au moins un polymère hydrophobiquement modifié de faible masse moléculaire en une quantité efficace pour inhiber l'entrée de virus dans des cellules,
dans laquelle ledit procédé comprend l'application de ladite composition antivirale à des surfaces infectables d'un sujet, lesdites surfaces infectables comprenant un ou plusieurs du groupe constitué par la peau et le tissu muqueux d'un sujet,
dans laquelle ledit polymère hydrophobiquement modifié de faible masse moléculaire comprend un polymère dérivé d'au moins un premier composant monomérique choisi dans le groupe constitué par un acide (méth)acrylique et au moins un deuxième composant monomérique choisi dans le groupe constitué par un ou plusieurs (méth)acrylates d'alkyle en C₁ à C₉,
dans laquelle le copolymère de faible masse moléculaire a une masse moléculaire moyenne en nombre de 100 000 Daltons ou moins, et
dans laquelle ladite composition est sensiblement exempte de tensioactif anionique, cationique ou amphotère présentant un équilibre hydrophile-lipophile (HLB) supérieur à 12.

2. Composition antivirale pour une utilisation selon la revendication 1, dans laquelle ledit tissu muqueux comprend un tissu choisi dans le groupe constitué par un tissu oral, un tissu oculaire, un tissu nasal, un tissu vaginal ou un tissu rectal et une combinaison de ceux-ci.

3. Composition antivirale pour une utilisation selon la revendication 1, dans laquelle ledit polymère hydrophobiquement modifié de faible masse moléculaire est présent dans ladite composition en une quantité de 0,00005 % à 10 % en poids de la composition.

4. Composition antivirale pour une utilisation selon la revendication 1, dans laquelle ladite composition comprend en outre au moins 50 % de solvant protique.

5. Composition antivirale pour une utilisation selon la revendication 4, dans laquelle ladite composition comprend au moins 97 % d'eau.

6. Composition antivirale pour une utilisation selon la revendication 1, dans laquelle lesdits virus sont choisis dans le groupe constitué par des poxviridae, herpesviridae, retroviridae Lentivirus et une combinaison de ceux-ci.

7. Composition antivirale pour une utilisation selon la revendication 6, dans laquelle :
(a) ledit virus choisi dans la famille des herpesviridae est le virus de l'herpès simplex 1, le virus de l'herpès simplex 2 et une combinaison de ceux-ci ; ou
(b) ledit virus choisi dans la famille des retroviridae Lentivirus est le virus d'immunodéficience humaine de type 1.

8. Composition antivirale pour une utilisation selon la revendication 1, dans laquelle :
(a) ladite inhibition de l'entrée du virus dans lesdites cellules entraînant la réduction du potentiel d'infection virale ; ou
(b) la composition antivirale ne perturbe pas sensiblement des surfaces biologiques.

9. Procédé pour inhiber la transmission de virus,
comprenant l'application sur des surfaces non biologiques d'une composition comprenant au moins un polymère hydrophobiquement modifié de faible masse moléculaire en une quantité efficace pour inhiber l'entrée de virus dans des cellules,
dans lequel ladite composition est sensiblement exempte de tensioactif anionique, cationique ou amphotère,
dans lequel ledit polymère hydrophobiquement modifié de faible masse moléculaire comprend un polymère dérivé d'au moins un premier composant monomérique choisi dans le groupe constitué par un acide (méth)acrylique et au moins un deuxième composant monomérique choisi dans le groupe constitué par un ou plusieurs (méth)acrylates d'alkyle en C₁ à C₉, et
dans lequel le copolymère de faible masse moléculaire a une masse moléculaire moyenne en nombre de 100 000 Daltons ou moins.

10. Procédé pour inhiber la transmission de virus,
comprenant l'application sur des surfaces ingérables d'une composition comprenant au moins un polymère hydrophobiquement modifié de faible masse moléculaire en une quantité efficace pour inhiber l'entrée de virus dans des cellules,
dans lequel ladite composition est sensiblement exempte de tensioactif anionique, cationique ou amphotère,
dans lequel ledit polymère hydrophobiquement modifié de faible masse moléculaire comprend un polymère dérivé d'au moins un premier composant monomérique choisi dans le groupe constitué par un acide (méth)acrylique et au moins un deuxième composant monomérique choisi dans le groupe constitué par un ou plusieurs (méth)acrylates d'alkyle en C₁ à C₉, et
dans lequel le copolymère de faible masse moléculaire a une masse moléculaire moyenne en nombre de 100 000 Daltons ou moins.

11. Composition antivirale pour une utilisation selon l'une quelconque des revendications 1 à 8, ou procédé selon la revendication 9 ou la revendication 10, dans laquelle/lequel ladite composition comprend une forme posologique choisie dans le groupe constitué par : un liquide, une lotion, une crème, un gel, un spray, une crème à raser, un onguent, un liquide nettoyant, un shampooing, une pâte, une mousse, un patch, et un hydrogel.

12. Composition antivirale pour une utilisation selon l'une quelconque des revendications 1 à 8 ou la revendication 11, ou procédé selon l'une quelconque des revendications 9 à 11, dans laquelle/lequel ledit polymère hydrophobiquement modifié de faible masse moléculaire comprend un copolymère acrylique linéaire non réticulé de faible masse moléculaire dérivé d'au moins un premier composant monomérique choisi dans le groupe constitué par un acide (méth)acrylique et d'au moins un deuxième composant monomérique choisi dans le groupe constitué par un ou plusieurs (méth)acrylates d'alkyle en C₁ à C₉, dans laquelle/lequel le copolymère de faible masse moléculaire a une masse moléculaire moyenne en nombre de 100 000 Daltons ou moins.

13. Composition antivirale pour une utilisation selon l'une quelconque des revendications 1 à 8, la revendication 11 ou la revendication 12, ou procédé selon l'une quelconque des revendications 9 à 12, dans laquelle/lequel ledit polymère hydrophobiquement modifié de faible masse moléculaire est un copolymère d'acrylates de potassium.

14. Composition antivirale pour une utilisation ou procédé selon la revendication 13, dans laquelle/lequel ladite composition comprend en outre du nonoxynol-9.
